# EUROPEAN PATENT SPECIFICATION

(11) **EP 3 777 787 B1**
(45) Date of publication and mention of the grant of the patent: **17.08.2022**
(21) Application number: 18912843.2
(22) Date of filing: 24.10.2018
(51) Int. Cl.: A61F 7/03, A61H 39/04, A61H 39/06, A61F 7/02

(54) **HEATING APPLIANCE**
HEIZGERÄT
APPAREIL DE CHAUFFAGE

(30) Priority: 27.03.2018 JP 2018060660
(43) Date of publication of application: 17.02.2021
(73) Proprietor: Kao Corporation, Chuo-ku Tokyo 103-8210 (JP)
(72) Inventor: ISHIKAWA, Shuji, Tokyo 131-8501 (JP)
(74) Representative: Hoffmann Eitle
(86) International application number: PCT/JP2018/039506
(87) International publication number: WO 2019/187278

(56) References cited:
- JP-A- H1 142 269
- JP-A- 2002 045 387
- JP-U- S5 552 262
- JP-U- S5 884 128
- JP-U- H02 141 449
- KR-A- 20150 145 848
- US-A1- 2004 055 076
- US-A1- 2009 222 072
- US-A1- 2018 021 168
- Khlystov Nikita ET AL: "Uniaxial Tension and Compression Testing of Materials", , 25 September 2013 (2013-09-25), pages 1-19, XP055790682, Retrieved from the Internet: URL:http://web.mit.edu/dlizardo/www/Uniaxi alTestingLabReportV6.pdf [retrieved on 2021-03-29]
- SMITH B H ET AL: "Steel foam for structures: A review of applications, manufacturing and material properties", JOURNAL OF CONSTRUCTIONAL STEEL RESEARCH, ELSEVIER, AMSTERDAM, NL, vol. 71, 29 October 2011 (2011-10-29), pages 1-10, XP028445687, ISSN: 0143-974X, DOI: 10.1016/J.JCSR.2011.10.028 [retrieved on 2011-11-04]

## Description

### TECHNICAL FIELD

The present invention relates to a heating implement.

### BACKGROUND ART

Japanese Patent Laid-Open No. 2005-111180 ("Patent Document 1") discloses a sheet-shaped heating implement. This heating implement is a sheet-shaped one molded by papermaking and has an entire surface configured to generate heat and a convex projection part on one side.

US 2004/0055076 A1 relates to an elastic textile belt for acupressure. The belt includes securing elements at opposite ends of the belt for securing the belt around the waist of a user; an abdominal acupressure element having a plurality of metal protrusions on the inner surface of the belt and a pouch for holding heat generating elements on an outer surface of the belt; a movable lumbar acupressure element having a plurality of metal protrusions on the inner surface, a plurality of hooks on the outer surface and a pouch for holding heat generating elements between the surfaces.

US 2009/0222072 A1 is directed to a reusable pain relieving treatment device, such as a belt, and a disposable thermal device having one or more thermally conductive components that extend from a surface of the device and are capable of transferring heat, cold or vibrations from disposable or reusable devices to targeted sections of the user's body.

JP-S-5884128 U relates to a heating element having an oxidizing exothermic substance such as iron powder inside, and a plurality of substantially similarly aligned hooklike projections such as a male body of a surface fastener on the surface, and a portable warming device comprising a mounting material which is freely attachable to the body and is a mounting surface.

JP 2002 045387 A relates to a band type body warmer comprising a heat generating body with an exothermic agent, wherein the heat generating body is intermittently covered by stretchable elastic regions.

### SUMMARY OF THE INVENTION

The present invention relates to a heating implement as recited in claim 1.

### BRIEF DESCRIPTION OF THE DRAWINGS

[Fig. 1] Fig. 1 is a plan view of a heating implement according to the first embodiment.
[Fig. 2] Fig. 2 is a cross-sectional view (a cross-sectional view taken along an A-A line of Fig. 1) of the heating implement according to the first embodiment.
[Fig. 3] Fig. 3 is an enlarged cross-sectional view of a projection part of the heating implement according to the first embodiment.

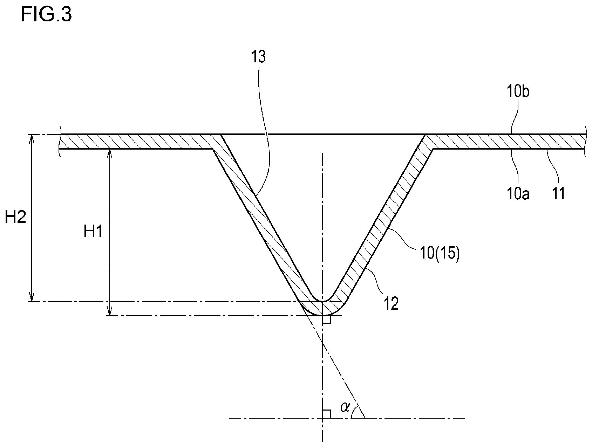

[Fig. 3] Fig. 3 is an enlarged cross-sectional view of a projection part of the heating implement according to the first embodiment.

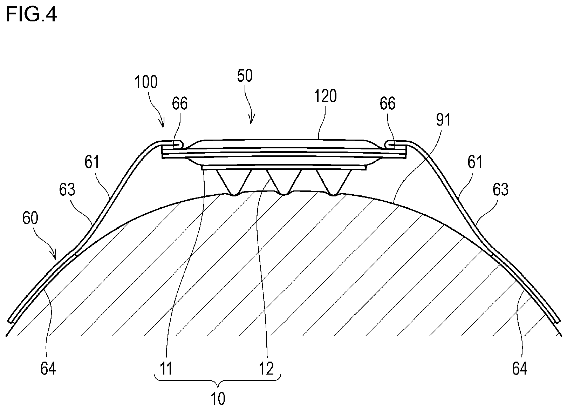

[Fig. 4] Fig. 4 is a schematic view showing a state where the heating implement according to the first embodiment is attached to a living body.

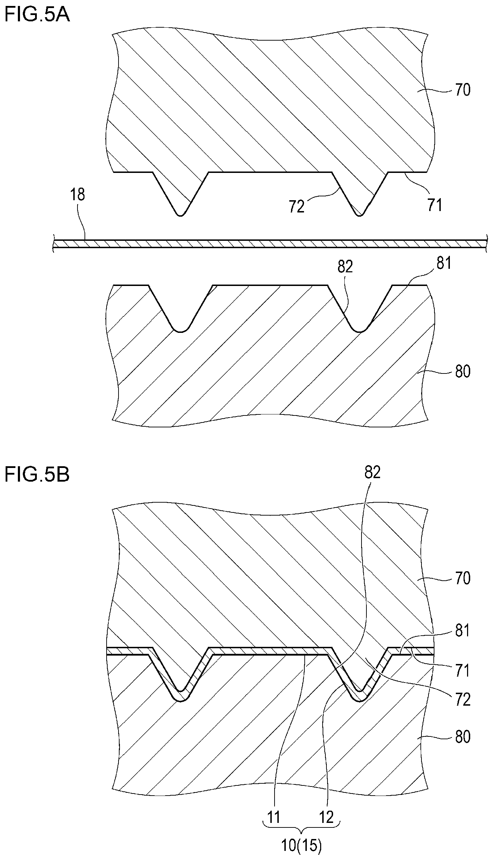

[Fig. 5] Figs. 5A and 5B are cross-sectional views showing a series of processes for forming the projection part on a sheet forming the heating implement according to the first embodiment.

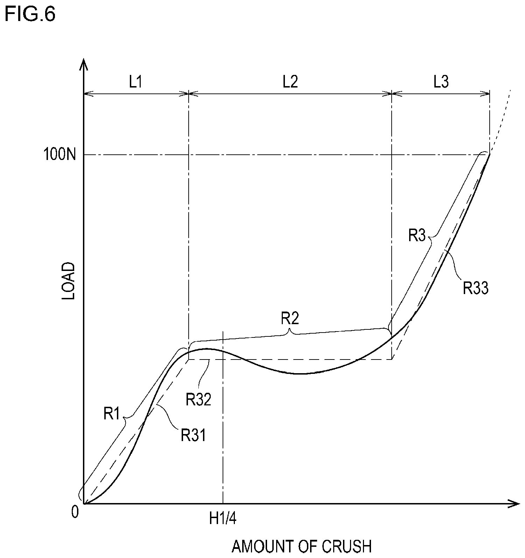

[Fig. 6] Fig. 6 is a view showing an example of a profile of a relationship between a load and an amount of crush on the projection part of the heating implement according to the first embodiment.

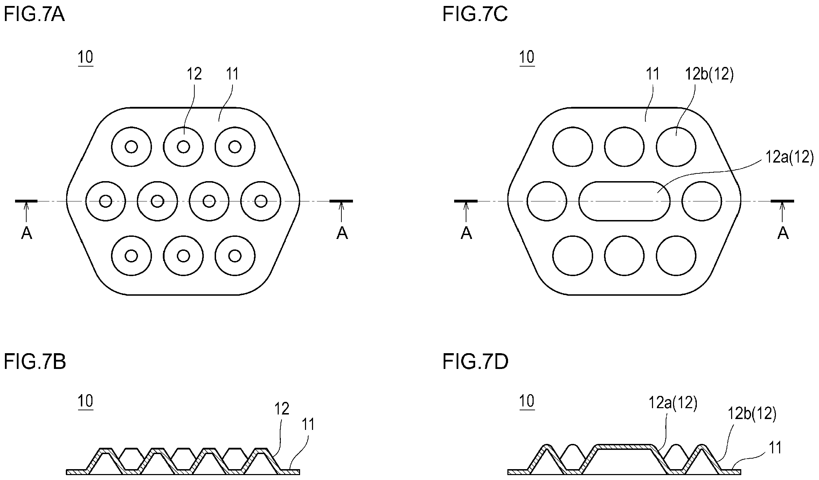

[Fig. 7] Figs. 7A, 7B, 7C, and 7D are views for explaining modified examples of a shape of the projection part of the heating implement according to the first embodiment.

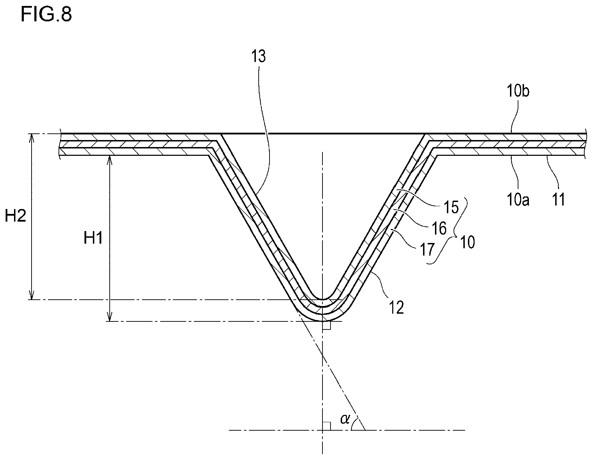

[Fig. 8] Fig. 8 is an enlarged cross-sectional view of the projection part of the heating implement according to the second embodiment.

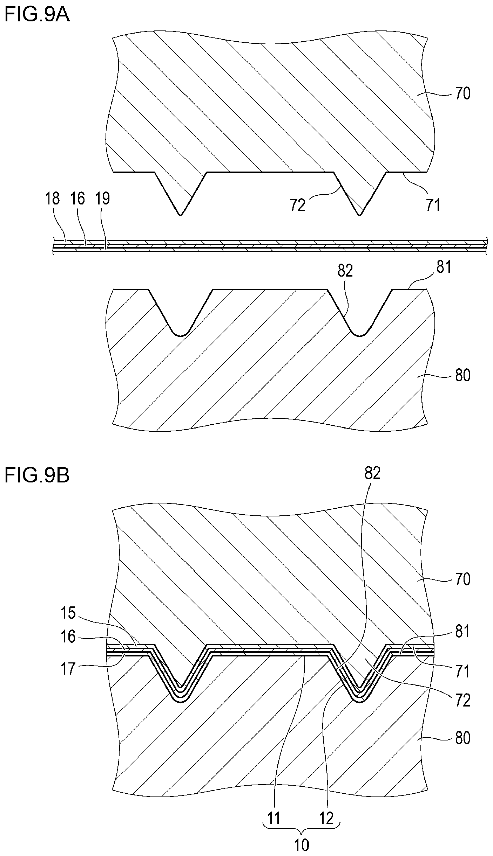

[Fig. 9] Figs. 9A and 9B are cross-sectional views showing a series of processes for forming the projection part on the sheet forming the heating implement according to the second embodiment.

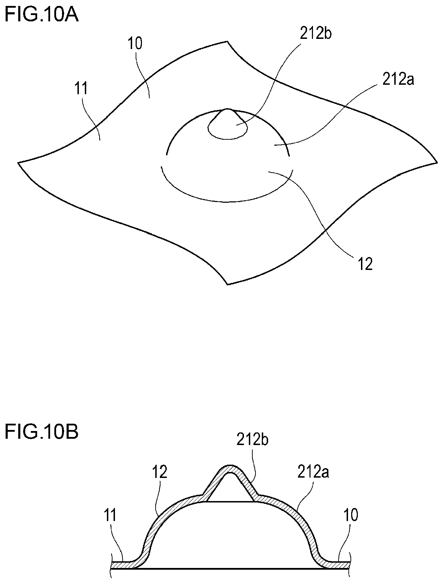

[Fig. 10] Figs. 10A and 10B are views showing the projection part of the heating implement according to the third embodiment, in which Fig. 10A is a perspective view and Fig. 10B is a cross-sectional view.

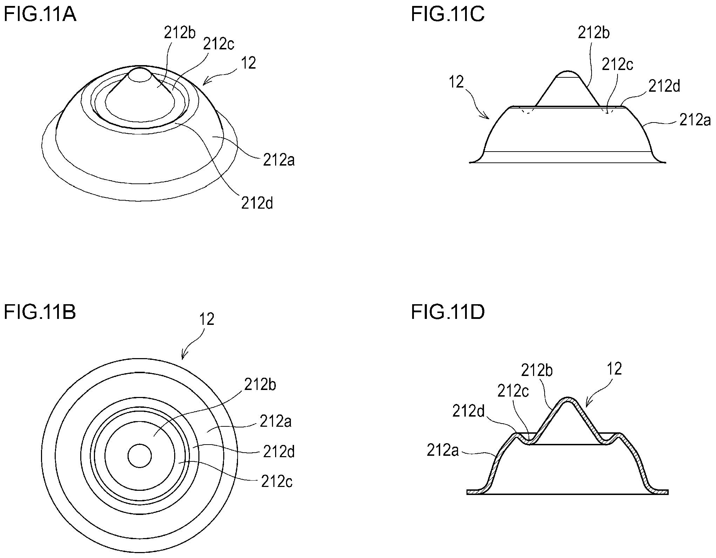

[Fig. 11] Figs. 11A, 11B, 11C, and 11D are views showing the projection part of the heating implement according to the fourth embodiment, in which Fig. 11A is a perspective view, Fig. 11B is a plan view, Fig. 11C is a side view, and Fig. 11D is a cross-sectional view.

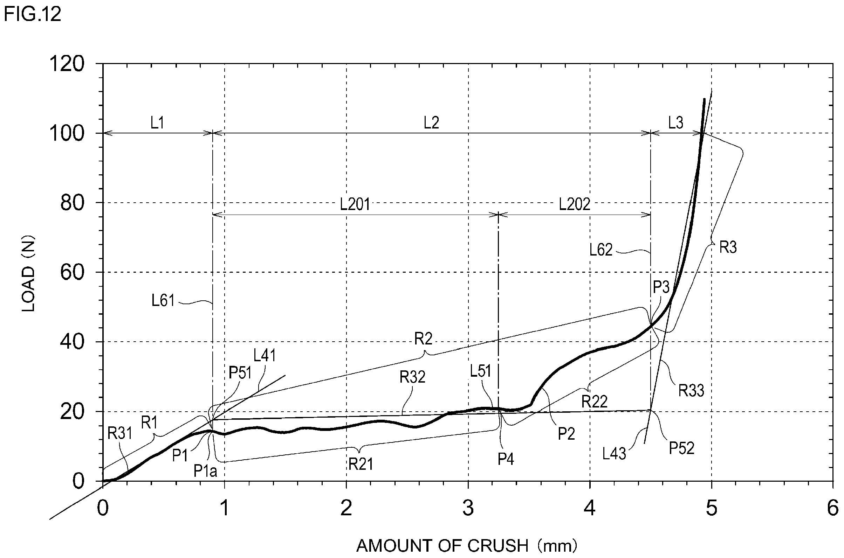

[Fig. 12] Fig. 12 is a view showing a profile of a relationship between a load and an amount of crush on a projection part of an example.

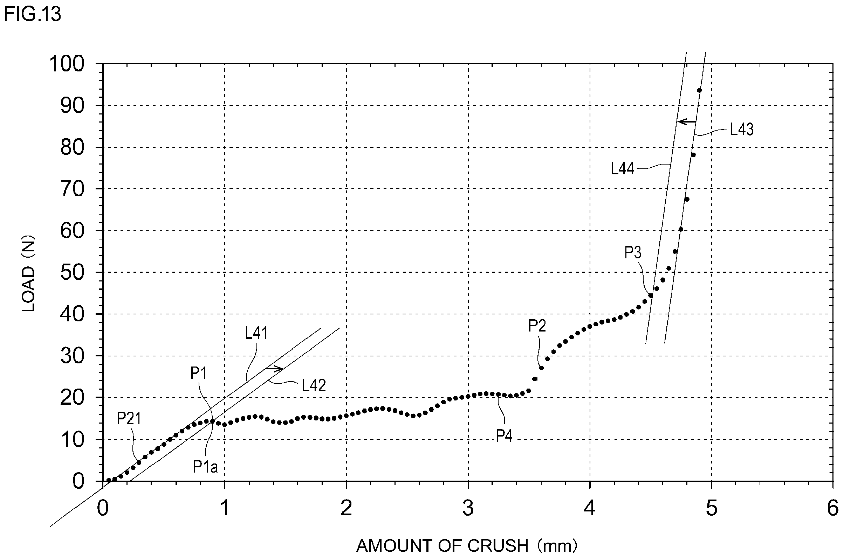

[Fig. 13] Fig. 13 is a view (only plot points) showing the profile of the relationship between the load and the amount of crush on the projection part of the example.

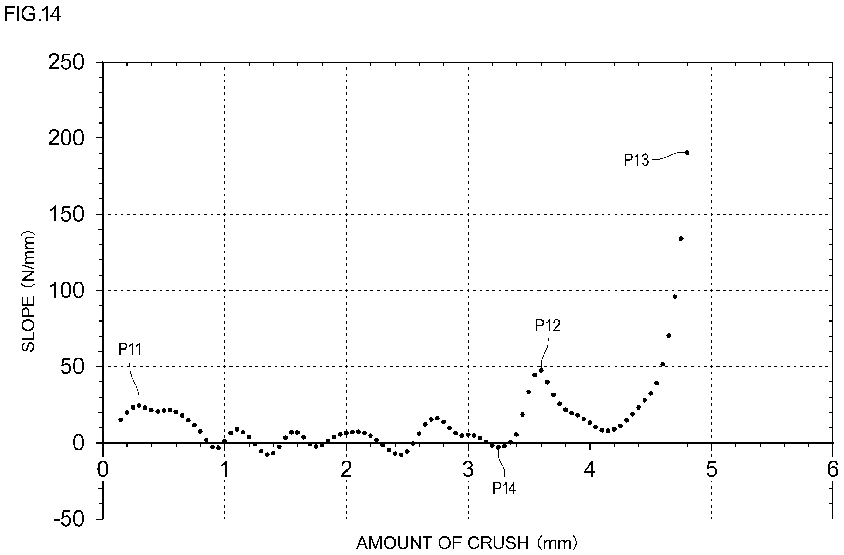

[Fig. 14] Fig. 14 is a view showing a slope of the profile of the relationship between the load and the amount of crush on the projection part of the example.

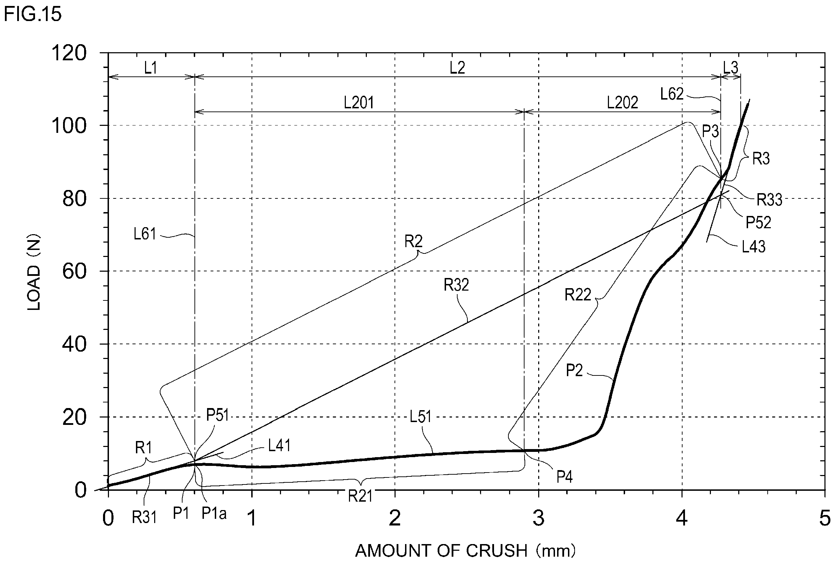

[Fig. 15] Fig. 15 is a view showing a profile of a relationship between a load and an amount of crush on a projection part of an example.

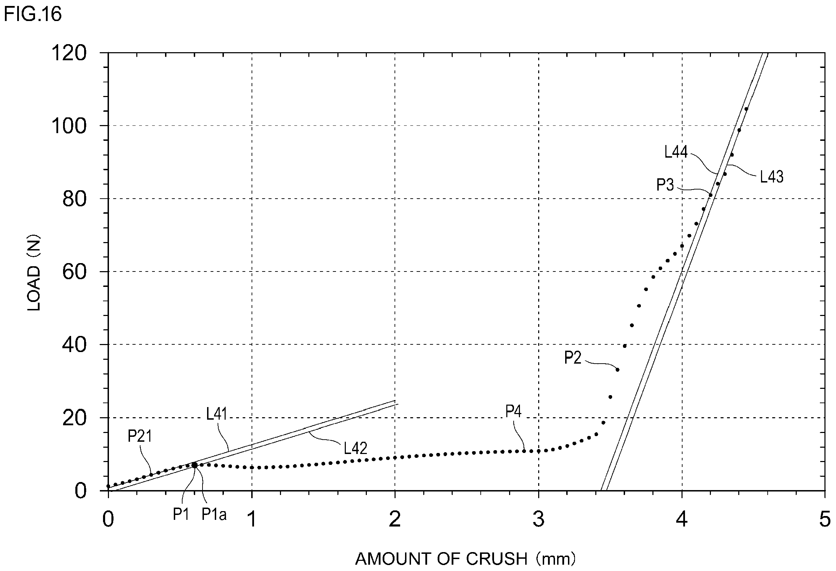

[Fig. 16] Fig. 16 is a view (only plot points) showing the profile of the relationship between the load and the amount of crush on the projection part of the example.

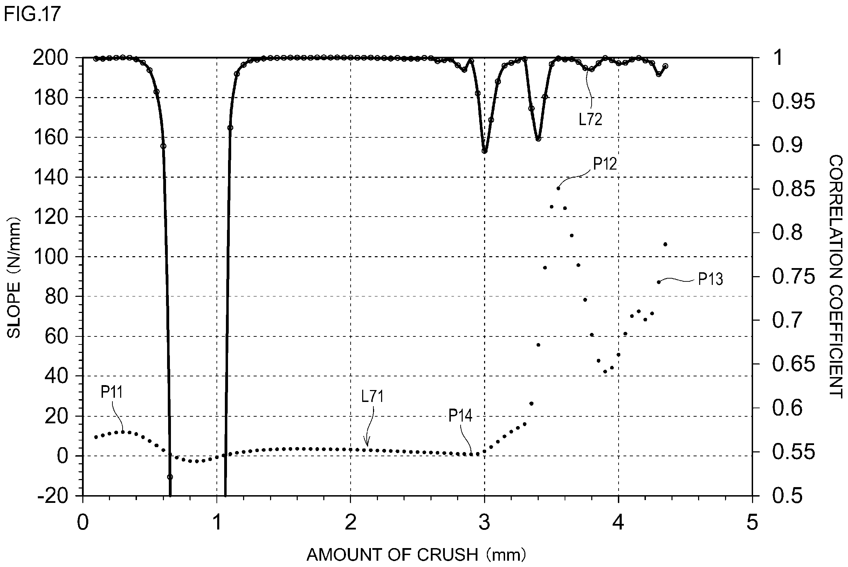

[Fig. 17] Fig. 17 is a view showing a slope of the profile of the relationship between the load and the amount of crush on the projection part of the example.

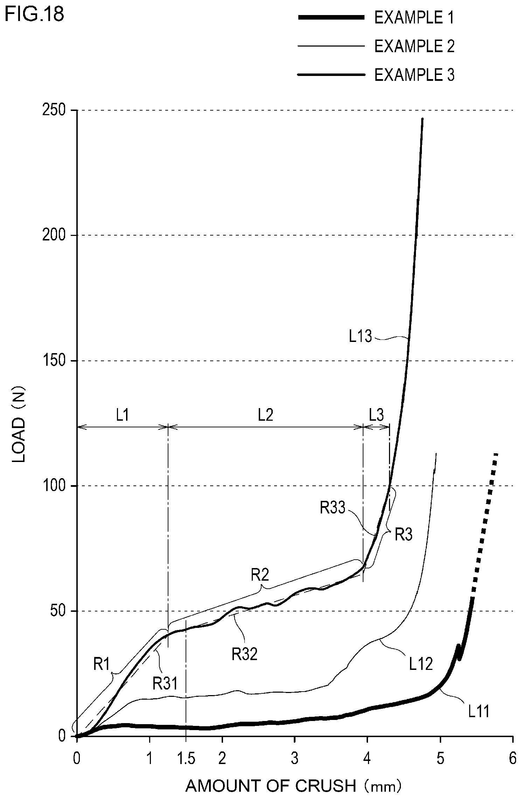

[Fig. 18] Fig. 18 is a view showing a profile of a relationship between a load and an amount of crush on projection parts of examples.

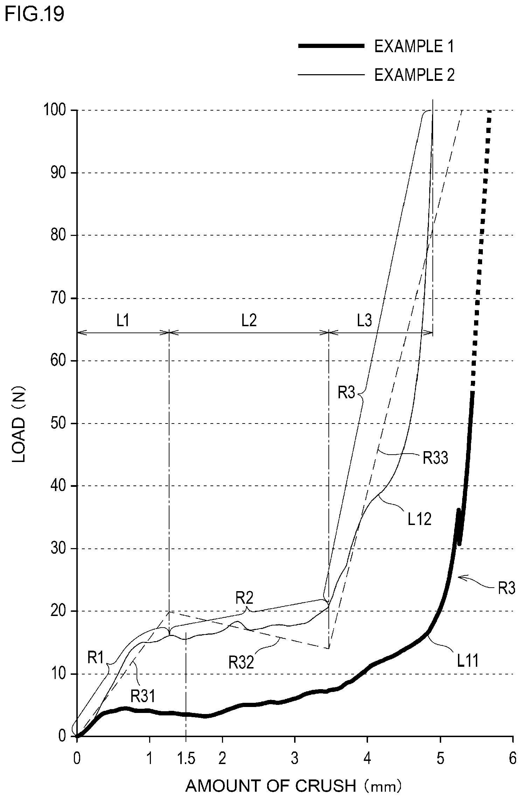

[Fig. 19] Fig. 19 is a view showing the profile of the relationship between the load and the amount of crush on the projection parts of the examples.

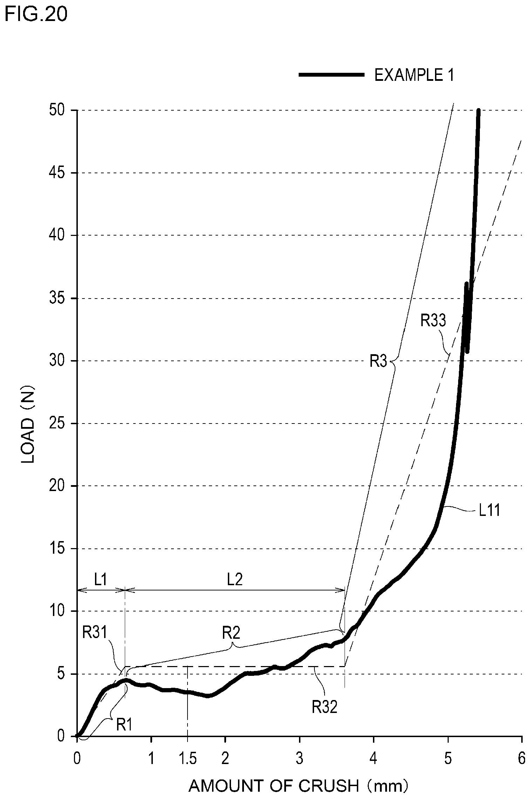

[Fig. 20] Fig. 20 is a view showing the profile of the relationship between the load and the amount of crush on the projection parts of the example.

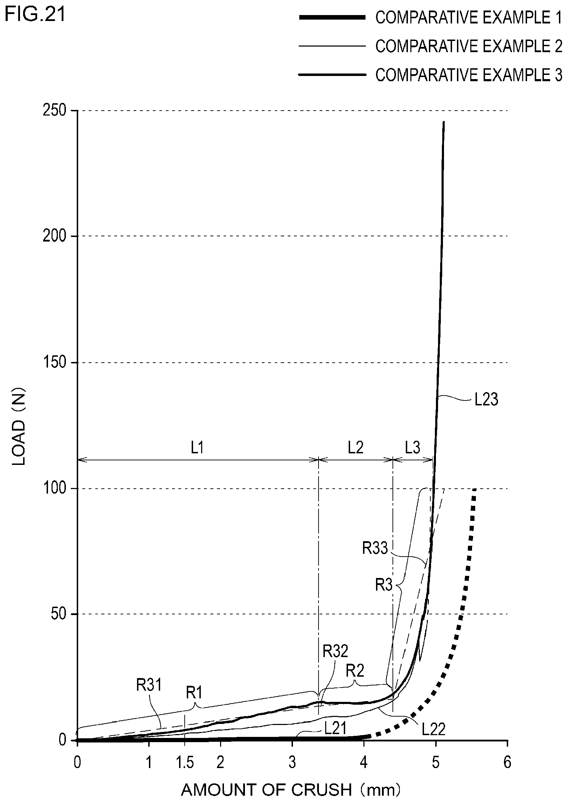

[Fig. 21] Fig. 21 is a view showing a profile of a relationship between a load and an amount of crush on projection parts of comparative examples.

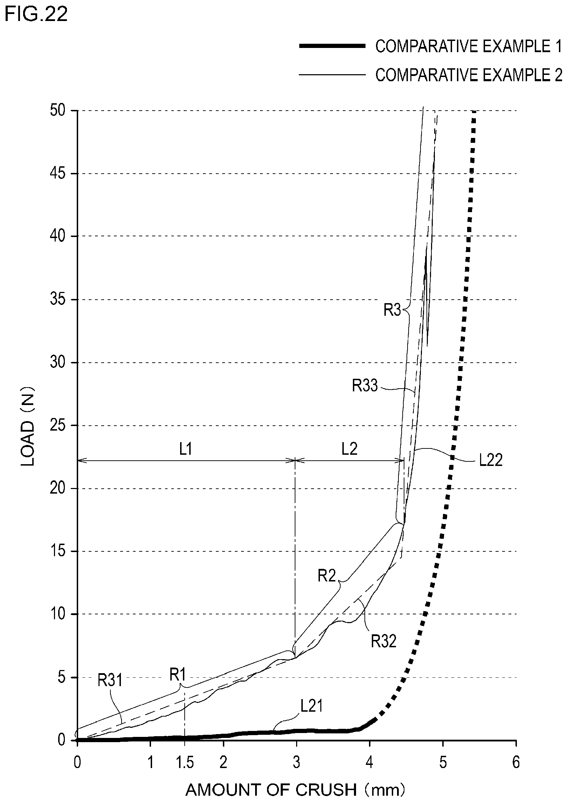

[Fig. 22] Fig. 22 is a view showing the profile of the relationship between the load and the amount of crush on the projection parts of the comparative examples.

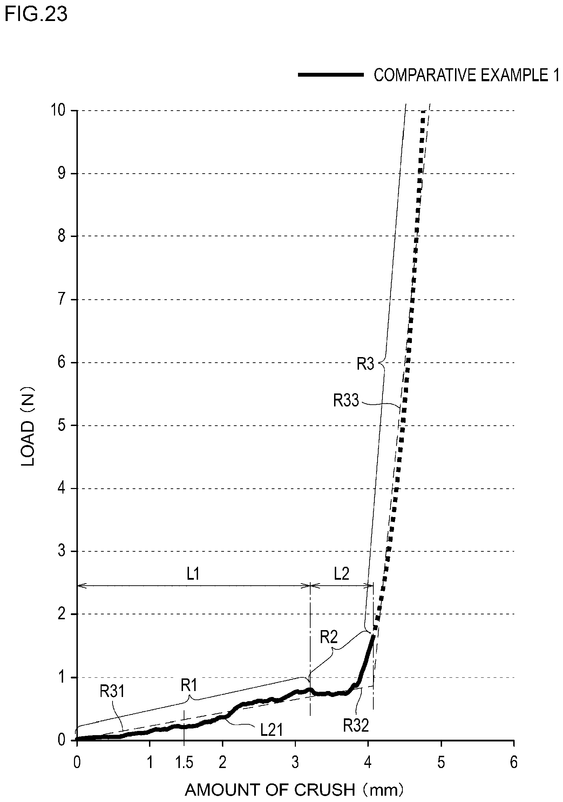

[Fig. 23] Fig. 23 is a view showing the profile of the relationship between the load and the amount of crush on the projection parts of the comparative example.

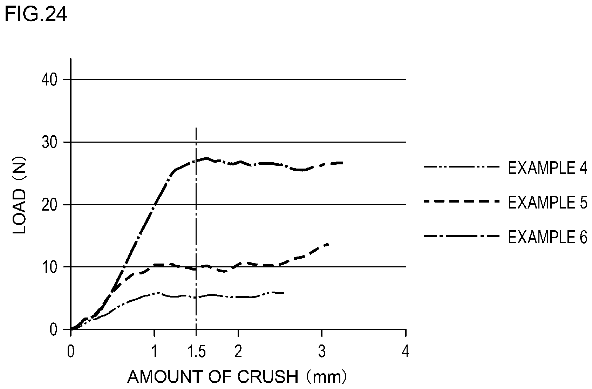

[Fig. 24] Fig. 24 is a view showing a profile of a relationship between a load and an amount of crush on projection parts of examples.

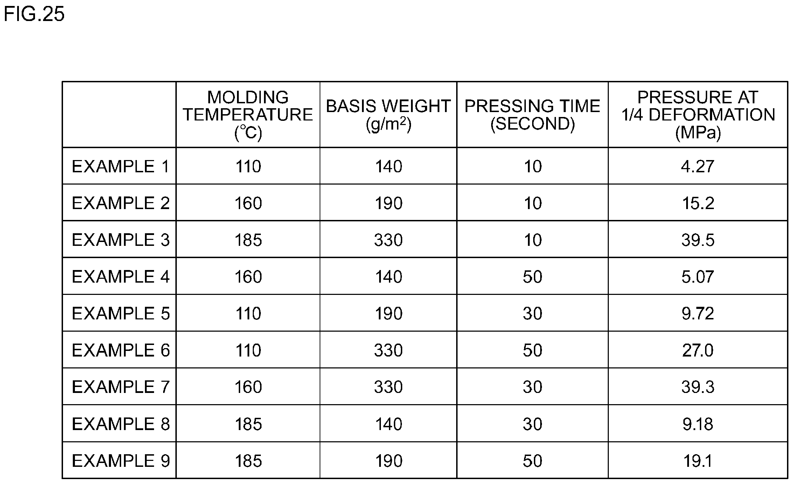

[Fig. 25] Fig. 25 is a view showing, for example, molding conditions of projection part sheets of examples.

### Detailed Description of the Invention

The heating implement of Patent Document 1 still has room for improvement in terms of, by the projection part, pressing a skin of a living body such as a human body in a more comfortable manner.

The present invention relates to a heating implement having a structure capable of, by a projection part, pressing a skin of a living body such as a human body in a more comfortable manner.

Hereinafter, preferred embodiments of the present invention will be explained with reference to the drawings. Note that in all the drawings, like components are marked with the same reference signs, and redundant explanations will not be repeated.

### [First embodiment]

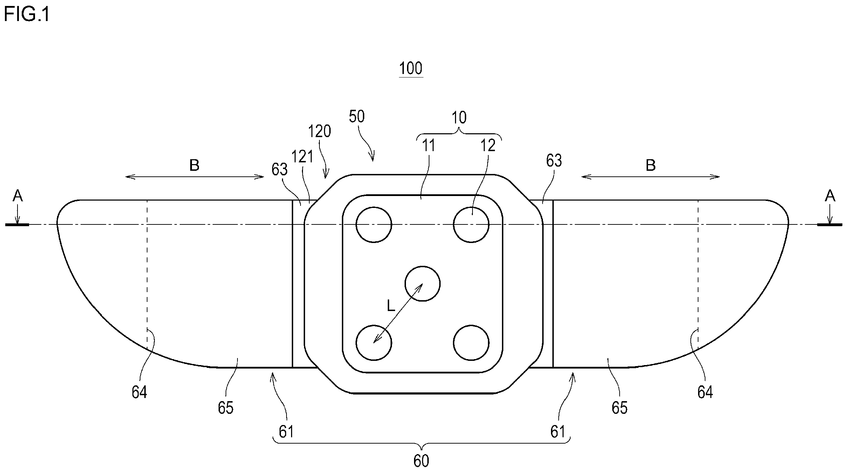

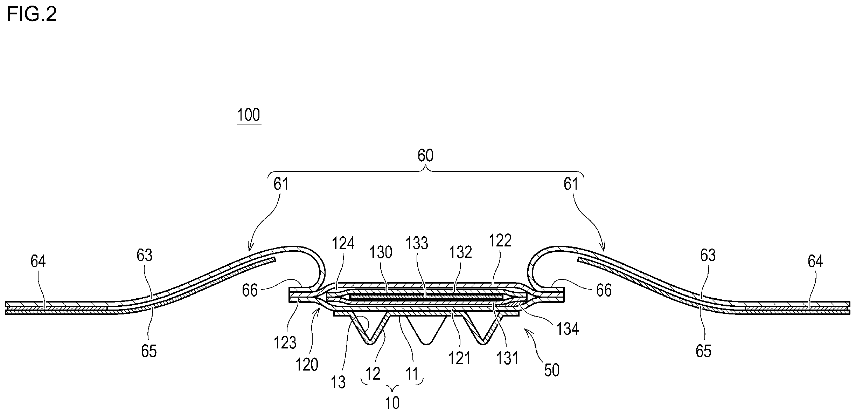

As shown in any of Figs. 1 to 3, a heating implement 100 according to the present embodiment includes a sheet-shaped main body sheet 120 having an exothermic element 130 (Fig. 2), and a projection part sheet 10 provided on a surface on one side of the main body sheet 120. The projection part sheet 10 has a projection part 12 projecting toward the above one side.

Assuming that a magnitude of a load when the projection part 12 is pressed in the direction opposite to the projecting direction of this projection part 12 is set to a first axis, and an amount of crush of this projection part 12 is set to a second axis, as shown in Fig. 6, a profile of a relationship between the above load and the above amount of crush includes a first region R1 in which the amount of crush increases as the load increases, and a second region R2 located on the side in which the value on the second axis is larger than that in the first region R1 (on the right side in Fig. 6) and having a larger increase rate of the amount of crush associated with increase in the load than the first region R1.

As shown in Fig. 6, when the above first axis is set to a vertical axis and the above second axis is set to a horizontal axis, the inclination angle of the above profile is gentler (smaller) in the second region R2 than in the first region R1.

Then, in the direction of the second axis (left-right direction in Fig. 6), the range of the second region R2 is wider than that of the first region R1. That is, in Fig. 6, a length L2 is longer than a length L1.

The heating implement 100 is attached to a living body such as a human body in a state where the projection part 12 is pressed against the skin, and thereby while the skin of the living body is pressed by the projection part 12, a surface of the living body can be warmed by heat of the exothermic element 130.

Thus, for example, pressure by the projection part 12 and stimulation by heat of the exothermic element 130 are applied to even an underlying fascia of the skin, so that the meridians and the acupuncture points can be stimulated by the pressure and the heat like acupuncture and moxibustion. That is, an effect of pressing pressure points can be obtained.

According to the heating implement 100 according to the present embodiment, the projection part 12 has the above described characteristic, and thereby when the load applied to this projection part 12 is less than a certain degree of magnitude (in the first region R1 of the above profile), a reaction force from the projection part 12 can be obtained while the projection part 12 is gradually crushed. On the other hand, when this load is equal to or more than a certain degree of magnitude (in the second region R2 of the above profile), the projection part 12 can be abruptly crushed. Thus, the reaction force from the projection part 12 can be prevented from becoming excessive, and accordingly a skin of a living body such as a human body can be pressed by the projection part 12 in a more comfortable manner.

Note that the deformation (crush) of the projection part 12 in the first region R1 in the above profile is considered to be caused by elastic deformation of the projection part 12. On the other hand, the deformation (crush) of the projection part 12 in the second region R2 in the above profile is considered to be caused by sudden crush of the projection part 12 by buckling of the projection part 12 due to application of the load exceeding the yield point or the yield strength point.

Fig. 6 shows an example of a profile of a relationship between the above load and the above amount of crush (smooth curve shown in Fig. 6) in the present embodiment.

As shown in Fig. 6, the increase rate of the amount of crush associated with increase in the load is larger in the second region R2 than in the first region R1.

Note that in the second region R2, the amount of crush may increase without substantial increase in the load, the amount of crush may increase while the load increases, or the amount of crush may increase while the load decreases.

As shown in Fig. 6, the above profile further includes a third region R3 located on the side in which the value on the second axis is larger than that in the second region R2 and having a smaller increase rate of the above amount of crush associated with increase in the above load than the second region R2.

As shown in Fig. 6, the inclination angle of the above profile is steeper (larger) in the third region R3 than in the second region R2.

Note that in the direction of the second axis (left-right direction in Fig. 6), no particular limitation is imposed on a magnitude relationship between the range of the second region R2 (length L2) and the range of the third region R3 (length L3), and a magnitude relationship between the range of the first region R1 (length L1) and the range of the third region R3 (length L3).

In the third region R3, the projection part 12 is substantially completely crushed, and the third region R3 is a region in which even when the load increases, further deformation of the projection part 12 slightly progresses or does not substantially progress.

Note that the first region R1 has a plurality of plot points except at both ends of this first region R1 and preferably has equal to or more than three plot points.

Similarly, the second region R2 has a plurality of plot points except at both ends of this second region R2 and preferably has equal to or more than three plot points.

Similarly, the third region R3 has a plurality of plot points except at both ends of this third region R3 and preferably has equal to or more than three plot points.

More specifically, for example, as shown in Fig. 6, when the above profile is approximated by three polygonal lines continuous with each other, a region corresponding to a first polygonal line portion R31 is the first region R1, a region corresponding to a second polygonal line portion R32 adjacent to the first polygonal line portion R31 is the second region R2, and a region corresponding to a third polygonal line portion R33 adjacent to the second polygonal line portion R32 is the third region R3.

Furthermore, as shown in Fig. 6, the above load is preferably 0 at one end of the first region R1. That is, the first region R1 is preferably a region from which application of the load to the projection part 12 starts.

Furthermore, as shown in Fig. 6, the third region R3 preferably has the above load within a range of equal to or less than 100 N.

Furthermore, as shown in Fig. 6, the second region R2 preferably includes a point in which the above amount of crush is 1/4 of a height of the projection part 12 (height dimension H1 (Fig. 4)).

Furthermore, the maximum value of the above load in the second region R2 is preferably equal to or less than 10 N, and also preferably equal to or less than 5 N. Thereby, a skin can be pressed by the projection part 12 in a more comfortable manner.

Furthermore, the above load at a boundary between the first region R1 and the second region R2 is preferably equal to or less than 20 N, also preferably equal to or less than 10 N, and also preferably equal to or less than 5 N. Thereby, a skin can be pressed by the projection part 12 in a more comfortable manner.

Furthermore, the minimum value of the above load in the second region R2 is preferably equal to or more than 0.2 N, and more preferably equal to or more than 0.4 N. Thereby, a skin can be pressed by the projection part 12 at a more sufficient strength.

More preferably, the plot interval (an interval between sampling values) of the above profile on the second axis is preferably equal to or more than 1/180 of the height dimension H1 of the projection part 12 and equal to or less than 1/100 thereof. That is, the sampling value corresponding to each of the plot points forming the profile is preferably, in the direction of the second axis, acquired (measured) at an interval of equal to or more than 1/180 of the height dimension H1 of the projection part 12 and equal to or less than 1/100 thereof.

An example of such a profile is shown in Fig. 12. Fig. 13 shows plot points forming the profile of Fig. 12, and a curve L51 shown in Fig. 12 is a profile obtained by connecting the plot points shown in Fig. 13.

The above profile is obtained by measuring both the load and the amount of crush while gradually applying a load to a projection part, and plotting a relationship between the two in a two-dimensional coordinate system. Thus, of the plot points forming the above profile, a plot point corresponding to the smallest amount of crush is referred to as a measurement start point, and a plot point corresponding to the largest amount of crush is referred to as a measurement end point.

Note that the plot points of the above profile are not necessarily limited to plot points that are actually measured, and may be plot points obtained by interpolation when the actually measured values are few.

Furthermore, the range of the above profile is preferably set to a range in which the load is equal to or less than 100 N. When the range of the above profile is set to the range in which the load is equal to or less than 100 N, of plot points in the range in which the load is equal to or less than 100 N, the measurement end point is a plot point corresponding to the largest amount of crush. However, as described above, the third region R3 is a region in which even when the load increases, further deformation of the projection part 12 slightly progresses or does not substantially progress, and accordingly a plot point measured until the projection part 12 is substantially completely crushed is preferably prepared.

### <Boundary point between the first region R1 and the second region R2>

A boundary point between the first region R1 and the second region R2 is preferably determined as follows.

First, the presence or absence of an upper yield point (described later) in the above profile is confirmed.

When the upper yield point exists, the upper yield point is set to the boundary point between the first region R1 and the second region R2.

When no upper yield point exists, a 1% yield strength point (described later) is set to the boundary point between the first region R1 and the second region R2.

### <Upper yield point>

When sampling values are sequentially evaluated from the measurement start point to the side in which the value on the second axis is large, it is confirmed whether sampling values in which the load does not change or the load decreases even when the amount of crush increases (sampling values such that the local slope of the above profile is zero or negative) consecutively appear.

When such sampling values consecutively appear, of those sampling values, a plot point corresponding to the first sampling value is set to the upper yield point. The upper yield point referred to here is set to an upper yield point existing in a region from the measurement start point to the occurrence of the amount of crush of 30% of the height dimension H1 of the projection part 12.

When such sampling values do not consecutively appear in the region (the region from the measurement start point to the occurrence of the amount of crush of 30% of the height dimension H1 of the projection part 12), it is assumed that no upper yield point exists.

In the examples of Figs. 13 and 12, a plot point P1 is the upper yield point, and this upper yield point is the boundary point between the first region R1 and the second region R2.

### <1% yield strength point>

Hereinafter, a method for obtaining the 1% yield strength point will be explained with the profiles of Figs. 12 and 13 as an example for convenience.

First, to evaluate the local slope of the above profile, an approximate straight line corresponding to each of local sections of the above profile is obtained by using a least squares method as explained below.

Of the sampling values from the measurement start point to the measurement end point, each of consecutive five sampling values is referred to as a unit sample group. Four sampling values are common between one unit sample group and the next unit sample group, and only one remaining sampling value is different therebetween. Furthermore, of the five sampling values of each unit sample group, a sampling value in which the amount of crush is the third is referred to as a center sampling value. Note that when there are less than five plot points that fall within the first region R1 as a result, these less than five plot points are set to a unit sample group, and of those, a sampling value in which the amount of crush is at the center position is set to the center sampling value.

For each unit sample group, the approximate straight line is obtained by the least squares method, and furthermore the slope (the load divided by the amount of crush) of each approximate straight line is obtained. In the present specification, the approximate straight line by the least squares method means an optimum straight line such that deviation between the value on the first axis (magnitude of the load) of each sampling value included in the unit sample group and this approximate straight line is minimized in the direction of the first axis.

For each unit sample group, a graph (Fig. 14) is created in which the value on the second axis of the center sampling value of this unit sample group and the slope of the approximate straight line corresponding to this unit sample group are plotted in a two-dimensional coordinate system. The horizontal axis of Fig. 14 corresponds to the horizontal axes (second axes) of Figs. 12 and 13, and the vertical axis of Fig. 14 shows the slope of each approximate straight line.

In the graph of Fig. 14, when evaluation is sequentially performed from plot points corresponding to the unit sample group on the side of the measurement start point, a plot point having the maximum value of this slope in a range in which the value of the amount of crush is smaller than the first minimum value of the value of the slope of the approximate straight line (hereinafter referred to as a first maximum plot point) is obtained. A plot point P11 of Fig. 14 is the first maximum plot point P11.

Then, the value on the vertical axis of the first maximum plot point P11, that is, the local slope of the above profile is set to an initial elastic modulus.

In the above profile (Figs. 12 and 13), a straight line passing through a plot point P21 (Fig. 13) corresponding to the first maximum plot point P11 and having a slope at the above initial elastic modulus is set to an approximate straight line L41 (Figs. 13 and 12) of the first region R1.

Next, an intersection point P1a between a 1% offset straight line L42 (Fig. 13) in which the approximate straight line L41 of the first region R1 is moved in parallel to the side in which the value on the second axis is large, by 1% of the height dimension H1 of the projection part 12, and the profile, is obtained.

This intersection point P1a is the 1% yield strength point. Then, the 1% yield strength point (intersection point P1a) is set to the boundary point between the first region R1 and the second region R2.

For example, as shown in Fig. 13, the upper yield point (plot point P1) and the 1% yield strength point (intersection point P1a) are present at positions substantially equal to each other or present at positions close to each other. Deviation between the plot point P1 and the intersection point P1a in the direction of the second axis is preferably equal to or less than the above plot interval.

### <Boundary point between the second region R2 and the third region R3>

When each plot point in the graph of Fig. 14 is sequentially evaluated from the measurement end point to the side in which the value on the second axis is small, a value in which the slope is largest in a range in which the amount of crush is larger than that at a point corresponding to the boundary point between the first region R1 and the second region R2 is referred to as a maximum inclination plot point P12.

In the graph of Fig. 14, a region between the maximum inclination plot point P12 and the measurement end point is focused on, and a correlation coefficient between each approximate straight line obtained by the least squares method and five sampling values included in the unit sample group corresponding to this approximate straight line is sequentially evaluated from the side of the measurement end point. Then, of approximate straight lines in which the correlation coefficient with five sampling values included in the corresponding unit sample group satisfies equal to or more than 90%, an approximate straight line that appears first (that is, of approximate straight lines in which this correlation coefficient satisfies equal to or more than 90%, an approximate straight line on the side closest to the measurement end point (on the right side)) is set to an approximate straight line L43 (Fig. 13) of the third region R3. Note that a plot point P13 of Fig. 14 is a plot point showing the magnitude of the slope of the approximate straight line L43 of the third region R3.

Here, in the range in which the load is equal to or less than 100 N, a case where no approximate straight line in which the above correlation coefficient satisfies equal to or more than 90% exists may also be considered. In that case, conversely, a plot point having the largest amount of crush in the range in which the load is equal to or less than 100 N is used as the starting point, and evaluation is sequentially performed to the side in which the amount of crush is larger. At this time, of approximate straight lines in which the correlation coefficient with five sampling values included in the corresponding unit sample group satisfies equal to or more than 90%, an approximate straight line that appears first is set to the approximate straight line of the third region R3.

Furthermore, an intersection point P3 (Figs. 13 and 12) between a 1% reverse offset straight line L44 (Fig. 13) in which the approximate straight line L43 of the third region R3 is moved to the direction in which the value on the second axis decreases (to the left side), by 1% of the height dimension H1 of the projection part 12, and the profile, is set to a boundary point between the second region R2 and the third region R3.

A straight line L61 shown in Fig. 12 is a straight line parallel to the first axis and passing through the plot point P1 (or intersection point P1a), and a straight line L62 is a straight line parallel to the first axis and passing through the intersection point P3.

In the profile of Fig. 12, the first polygonal line portion R31 is a part of the approximate straight line L41. That is, the first polygonal line portion R31 is a portion from a point in which, in the approximate straight line L41, the amount of crush corresponds to 0 to an intersection point P51 between the approximate straight line L41 and the straight line L61.

In the profile of Fig. 12, the third polygonal line portion R33 is a part of the approximate straight line L43. That is, the third polygonal line portion R33 is a portion from an intersection point P52 between the approximate straight line L43 and the straight line L62 to a point in which the load first becomes 100 N.

In the profile of Fig. 12, the second polygonal line portion R32 is a line segment connecting the intersection point P51 between the approximate straight line L41 and the straight line L61 and the intersection point P52 between the approximate straight line L43 and the straight line L62.

Here, the second region R2 can be divided into two regions by using a second region division point P4 shown in Fig. 12 as the boundary point. Of these two regions, a region on the side in which the value on the second axis is small is referred to as a second region former half part R21 (Fig. 12), and a region on the side in which the value on the second axis is large is referred to as a second region latter half part R22 (Fig. 12).

The second region division point P4 can be obtained as follows.

In the graph of Fig. 14, between a portion corresponding to the boundary point between the second region and the first region and a portion corresponding to the boundary point between the third region and the second region, a value in which the slope is largest is obtained. In the example of Fig. 14, this value is the maximum inclination plot point P12. When the maximum inclination plot point P12 is used as the starting point, and evaluation is sequentially performed from plot points corresponding to the unit sample group in which the amount of crush is largest to the side in which the amount of crush is small (that is, the negative side on the second axis), a plot point that is the first minimum value is set to a minimum plot point P14. In the profiles of Figs. 12 and 13, a point corresponding to the minimum plot point P14 is the second region division point P4.

Assuming that the load at the boundary point between the first region R1 and the second region R2 (plot point P1 or intersection point P1a) is set to F1, and the load at the boundary point between the second region former half part R21 and the second region latter half part R22 (second region division point P4) is set to F2, 0.8 < (F2/F1) ≤ 3 is preferably satisfied, and (F2/F1) ≤ 2 is more preferably satisfied. Thus, the projection part 12 can be more abruptly crushed at the second region former half part R21 of the above profile, and accordingly the reaction force from the projection part 12 can be further prevented from becoming excessive, so that a skin of a living body such as a human body can be pressed by the projection part 12 in a more comfortable manner.

Furthermore, in the direction of the second axis, the range of the second region former half part R21 is preferably wider than that of the second region latter half part R22. That is, in Fig. 12, a length L201 is preferably longer than a length L202. Thus, a range of the amount of crush in which a skin of a living body such as a human body can be pressed by the projection part 12 in a comfortable manner can be more sufficiently ensured.

Furthermore, the slope of the second region latter half part R22 (the slope of the line segment connecting the start point of the second region latter half part R22 and the end point thereof) is preferably positive, and the absolute value of the slope of the second region former half part R21 (the slope of the line segment connecting the start point of the second region former half part R21 and the end point thereof) is preferably smaller than the value of the slope of the second region latter half part R22. That is, the second region former half part R21 is preferably closer to horizontal than the second region latter half part R22.

In this way, in the range in which the load is equal to or less than 100 N, the above profile includes, in the direction of the second axis, the plot point plotted at the plot interval of equal to or more than 1/180 of the height dimension of the projection part 12 and equal to or less than 1/100 thereof. Of the sampling values from the measurement start point to the measurement end point, each of consecutive five sampling values is set to the unit sample group, and of the five sampling values of the unit sample group, the sampling value in which the amount of crush is the third is set to the center sampling value. For each unit sample group, the slope of the approximate straight line obtained by the least squares method is obtained, and the graph (Fig. 14) in which the obtained slope and the amount of crush of each center sampling value are plotted in a two-dimensional coordinate system is obtained. In the graph, at the region in which the amount of crush is smaller than that at the point corresponding to the boundary point between the third region R3 and the second region R2 (intersection point P3), and the amount of crush is larger than that at the point corresponding to the boundary point between the second region R2 and the first region R1 (plot point P1 or intersection point P1a), the plot point having the maximum value of the slope of the approximate straight line is set to the maximum inclination plot point P12. Furthermore, when the maximum inclination plot point P12 is used as the starting point, and evaluation is sequentially performed from the plot points corresponding to the unit sample group in which the amount of crush is largest to the side in which the amount of crush is small, the plot point having the first minimum value of the slope of the approximate straight line is set to the minimum plot point P14. Assuming that the load at the boundary point between the first region R1 and the second region R2 (plot point P1 or intersection point P1a) is set to F1, and the load at the point (second region division point P4) corresponding to the minimum plot point P14 in the above profile is set to F2, 0.8 < (F2/F1) ≤ 3 is preferably satisfied.

Furthermore, when the second region division point P4 (Figs. 13 and 12) corresponding to the minimum plot point P14 is set to the boundary point, and the second region R2 is divided into two regions of the second region former half part R21 located on the side in which the value on the second axis is small and the second region latter half part R22 located on the side in which the value on the second axis is large, in the direction of the second axis, the range (length L201 shown in Fig. 12) of the second region former half part R21 is preferably wider than that (length L202 shown in Fig. 12) of the second region latter half part R22.

In the case of the present embodiment, the projection part 12 has air permeability. More specifically, the projection part sheet 10 including the projection part 12 as a whole has air permeability. Thus, the heat of the exothermic element 130 can be transmitted to a skin through the projection part 12 in an improved manner. In particular, when the exothermic element 130 generates vapor, the heat of the exothermic element 130 can be transmitted to a skin through the projection part 12 in a more improved manner.

The projection part sheet 10 includes a nonwoven sheet 15 (Fig. 3).

In the case of the present embodiment, the projection part sheet 10 is formed of one layer of the nonwoven sheet 15. The nonwoven sheet 15 includes fibers formed of a first resin material, and a binding part formed of a second resin material having a lower melting point than the first resin material and binding together the fibers.

Thus, rigidity of the projection part sheet 10 and consequently rigidity of the projection part 12 of the projection part sheet 10 can be sufficiently ensured. Accordingly, a skin of a living body such as a human body can be sufficiently pressed by the projection part 12.

Note that the projection part sheet 10 and the nonwoven sheet 15 may further include a second binding part. The second binding part is formed of at least equal to or more than one resin material having a lower melting point than the first resin material and a higher melting point than the second resin material and binding together fibers of a resin material (a resin group (including at least the first resin material) that does not melt during the processing of a nonwoven fabric associated with the molding of the projection part 12) having a higher melting point than this resin material.

In the case of the present embodiment, the content of the first resin material in the nonwoven sheet 15 is larger than that of the second resin material in the nonwoven sheet 15.

Thus, the rigidity of the projection part sheet 10 can be in a suitable range (not too hard). Furthermore, the air permeability of the projection part sheet 10 can be easily ensured.

The projection part sheet 10 includes, for example, a flat sheet-shaped base part 11, and the projection part 12 curving convexly on the side of a surface on one side 10a of the projection part sheet 10 with the base part 11 as a reference and having a cavity 13 on the side of a surface on the other side 10b.

In the case of the present embodiment, no solid matter (solid) or liquid is filled in the cavity 13, and the inside of the cavity 13 is hollow.

In the case of the present embodiment, in the heating implement 100, a portion including the above main body sheet 120 and the projection part sheet 10 is referred to as a main body 50. The main body 50 is applied to a portion of a skin of a living body to which heat is desired to be provided.

The main body sheet 120 includes a first sheet 121 located on the skin side of a user in a state where the main body 50 is attached to the user, and a second sheet 122 located on the side opposite to the skin side of the user in the state where the main body 50 is attached to the user. The first sheet 121 and the second sheet 122 are superimposed on each other.

The first sheet 121 and the second sheet 122 are joined to each other at, for example, an annular joint part 123 located at peripheral edge portions thereof. The first sheet 121 and the second sheet 122 may be joined by adhesion or bonding or may be joined by heat sealing.

Each of the first sheet 121 and the second sheet 122 may be formed of a single layer of a sheet or may be a laminate of a plurality of sheets.

Examples of the materials of the sheet members (first sheet 121 and second sheet 122) forming the main body sheet 120 include a nonwoven fabric, a woven fabric, another knitted fabric, a resin film of polyethylene, urethane, or the like, a porous body, and any combination of equal to or more than two kinds of them.

The projection part sheet 10 is attached to the surface on the one side of the main body sheet 120, that is, an outer surface of the first sheet 121. The projecting direction of the projection part 12 from the base part 11 is opposite to the side of the main body sheet 120.

A gap between the first sheet 121 and the second sheet 122, that is, a region surrounded by the annular joint part 123 is an accommodation space 124 that accommodates the exothermic element 130.

The exothermic element 130 includes, for example, a first covering sheet 131, a second covering sheet 132, and a sheet-shaped exothermic part 133 held between the first covering sheet 131 and the second covering sheet 132.

The form of the exothermic part 133 is not particularly limited, but examples thereof include three types of a coating type, a powder type, and a papermaking (sheet forming) type.

Of these, the coating-type exothermic part 133 is configured by applying an exothermic composition (an exothermic composition including iron powder, activated carbon, water, and the like) that can be applied to crepe paper or a laminate of paper, and laminating a polymer sheet thereon. Instead of the polymer sheet, a water absorbing polymer or a water absorbing layer such as paper or a rayon nonwoven fabric may be used.

The powder-type exothermic part 133 is configured by compressing powder obtained by mixing iron, activated carbon, water, a super absorbent polymer (SAP), inorganic powder, and the like into a sheet, and enclosing this between the first covering sheet 131 and the second covering sheet 132.

The papermaking-type exothermic part 133 is configured by adding saline to an exothermic material including iron powder, activated carbon, and pulp, and enclosing this between the first covering sheet 131 and the second covering sheet 132.

The first covering sheet 131 and the second covering sheet 132 are superimposed on each other. Thus, the first covering sheet 131 and the second covering sheet 132 form an accommodation body that accommodates the exothermic part 133 inside.

The first covering sheet 131 and the second covering sheet 132 are joined to each other at, for example, peripheral edge portions thereof.

The first covering sheet 131 and the second covering sheet 132 may be joined by adhesion or bonding or may be joined by heat sealing.

Of the first covering sheet 131 and the second covering sheet 132, the first covering sheet 131 is disposed on the side of the first sheet 121, that is, the skin side of the user in a state where the main body 50 is attached, and the second covering sheet 132 is disposed on the side of the second sheet 122, that is, the side opposite to the skin side of the user in the state where the main body 50 is attached.

Note that the present invention is not limited to this example, and the exothermic element 130 may not have the first covering sheet 131 and the second covering sheet 132. In this case, the accommodation body that accommodates the exothermic part 133 inside is formed of, for example, the first sheet 121 and the second sheet 122. Furthermore, in this case, the first sheet 121 has a function of the first covering sheet 131 (for example, air permeability of the first covering sheet 131), and the second sheet 122 has a function of the second covering sheet 132 (for example, air permeability of the second covering sheet 132).

At least a part of an outer surface of the exothermic element 130 is joined to an inner surface of the main body sheet 120 at a joint part 134.

At least one of the first covering sheet 131 and the second covering sheet 132 is formed of a material having air permeability. In the case of the present embodiment, the first covering sheet 131 has a higher air permeability than the second covering sheet 132. Note that the second covering sheet 132 may have air permeability or may not substantially have air permeability.

Furthermore, the first covering sheet 131 is a moisture permeable sheet. On the other hand, the second covering sheet 132 is a moisture permeable sheet or moisture impermeable sheet. When the second covering sheet 132 is a moisture permeable sheet, air permeability of this second covering sheet 132 may be the same as that of the first covering sheet 131, may be lower than that of the first covering sheet 131, or may be higher than that of the first covering sheet 131.

Furthermore, air permeability of the first sheet 121 is preferably higher than that of the first covering sheet 131, and air permeability of the second sheet 122 is preferably higher than that of the second covering sheet 132. When the second sheet 122 is air impermeable, the second covering sheet 132 may be air impermeable, or the second covering sheet 132 may not be air impermeable.

Note that when the air permeability of the second covering sheet 132 is lower than that of the first covering sheet 131, it is easier to release vapor to the skin side.

Furthermore, the first sheet 121 is formed of a material having air permeability and moisture permeability. The second sheet 122 may have air permeability or may not substantially have air permeability. Furthermore, the second sheet 122 may have moisture permeability or may not substantially have moisture permeability.

Note that the air permeability of the projection part sheet 10 is preferably higher than that of the first sheet 121.

More specifically, in the case of the present embodiment, the second sheet 122 is, for example, an air impermeable sheet which is substantially impermeable to air.

Note that the heating implement 100 is, in a pre-use state, accommodated in a packaging material, which is not shown in the drawings, in an airtight manner. When the packaging material is opened and the heating implement 100 is taken out of the packaging material, oxygen included in the outside air is supplied to the exothermic element 130, so that this exothermic element 130 generates heat.

Note that, as described above, the projection part 12 and consequently the projection part sheet 10 as a whole have air permeability, and accordingly oxygen can be supplied to the exothermic part 133 of the exothermic element 130 via the projection part sheet 10, the first sheet 121, and the first covering sheet 131.

The heating implement 100 is taken out of the packaging material when using and thereby the exothermic material inside the exothermic part 133 of the exothermic element 130 is brought into contact with oxygen in the air. Then, the exothermic part 133 generates heat and generates water vapor (vapor heat), and this water vapor is released to the outside via the first covering sheet 131, the first sheet 121, and the projection part sheet 10.

Accordingly, the heat of the exothermic element 130 can be quickly transmitted to a skin of a living body by latent heat of the water vapor.

The projection part 12 also has air permeability, and thereby heat can be transmitted to a skin by water vapor released from the projection part 12. Accordingly, while the skin is heated by the projection part 12, the skin can be pressed by the projection part 12.

Here, the degree of air permeability of the projection part sheet 10 is preferably equal to or more than 1 second/100 ml, and more preferably equal to or more than 3 seconds/100 ml. Furthermore, it is preferably equal to or less than 20000 seconds/100 ml, and more preferably equal to or less than 10000 seconds/100 ml.

Furthermore, the degree of air permeability of the nonwoven sheet 15 is preferably equal to or more than 1 second/100 ml, and more preferably equal to or more than 3 seconds/100 ml. Furthermore, it is preferably equal to or less than 20000 seconds/100 ml, and more preferably equal to or less than 10000 seconds/100 ml.

Furthermore, the degree of air permeability of the first sheet 121 is preferably equal to or less than 20000 seconds/100 ml, and more preferably equal to or less than 10000 seconds/100 ml.

Furthermore, the degree of air permeability of the second sheet 122 is preferably the same as or higher than that of the second covering sheet 132.

The degree of air permeability is a value measured according to JIS P8117 and is defined by a time in which 100 ml of air passes through an area of 6.45 cm² under a constant pressure. The degree of air permeability can be measured by an Oken type air permeability meter or a measuring instrument according thereto.

In the present specification, having air permeability means that the degree of air permeability is equal to or less than 190000 seconds/100 ml, and preferably equal to or less than 100000 seconds/100 ml. Furthermore, being air impermeable means that the degree of air permeability exceeds 190000 seconds/100 ml.

The planar shape of the main body sheet 120 is not particularly limited but can be in, for example, a rectangular shape. Alternatively, the planar shape of the main body sheet 120 may be in a polygonal shape other than a rectangle or in another shape such as a circle or an ellipse.

Furthermore, the planar shape of the projection part sheet 10 is not particularly limited but can be in, for example, a rectangular shape (for example, a square shape) whose each of four corner portions is in a chamfered shape as shown in Fig. 1.

In the case of the present embodiment, the main body 50 including the main body sheet 120 and the projection part sheet 10 has, for example, a rectangular shape.

In the following explanation, the projecting direction of the projection part 12 (downward in Fig. 2) in the main body 50 may be referred to as a front side, and the direction opposite to the projecting direction of the projection part 12 (upward in Fig. 2) may be referred to as a rear side.

The projection part sheet 10 and the first sheet 121 of the main body sheet 120 form an outer surface on the front side of the main body 50. For example, in a state where the projection part sheet 10 (in particular, the projection part 12) is directly in contact with a skin of a living body, the heating implement 100 is used.

Furthermore, the second sheet 122 of the main body sheet 120 forms an outer surface on the rear side of the main body 50.

The shape of the projection part 12 is not particularly limited but is, for example, a shape tapered toward the tip side. Additionally, a tip portion of the projection part 12 preferably has a rounded shape.

The shape of the projection part 12 can be, for example, a cone shape such as a circular cone shape, an elliptic cone shape, or a long circular cone shape, or a truncated cone shape such as a truncated circular cone shape, a truncated elliptic cone shape, or a truncated long circular cone shape.

In the case of the present embodiment, the shape of the projection part 12 is formed in a circular cone shape.

The height dimension H1 (Fig. 3) of the projection part 12 is not particularly limited but is, for example, preferably equal to or more than 2 mm and equal to or less than 15 mm, more preferably equal to or more than 3 mm and equal to or less than 10 mm, and still more preferably equal to or more than 5 mm and equal to or less than 8 mm.

The height dimension H1 of the projection part 12 is equal to or more than 2 mm and equal to or less than 15 mm, and thereby a skin of a living body can be sufficiently and suitably pressed by the projection part 12.

The diameter of the projection part 12 is not particularly limited but is, for example, preferably equal to or more than 2 mm and equal to or less than 38 mm, and more preferably equal to or more than 5 mm and equal to or less than 20 mm. The diameter of the projection part 12 is equal to or more than 2 mm and equal to or less than 38 mm, and thereby a skin of a living body can be sufficiently and suitably pressed by the projection part 12.

An inclination angle α (Fig. 3) of a side surface of the projection part 12 is not particularly limited but is, for example, preferably equal to or more than 30 degrees, and more preferably equal to or more than 45 degrees. The inclination angle α of the projection part 12 is equal to or more than 30 degrees, and thereby a skin of a living body can be sufficiently pressed by the projection part 12.

Furthermore, the inclination angle α of the side surface of the projection part 12 is preferably equal to or less than 80 degrees, more preferably equal to or less than 70 degrees, and still more preferably equal to or less than 65 degrees. The inclination angle α of the projection part 12 is equal to or less than 80 degrees, and thereby the degree of penetration of the projection part 12 into a skin of a living body can be within a suitable range.

Note that, as described above, the tip portion of the projection part 12 preferably has a rounded shape. The radius of curvature of the tip portion of the projection part 12 is preferably equal to or more than 0.5 mm and equal to or less than 3.0 mm, and more preferably equal to or more than 0.8 mm and equal to or less than 1.5 mm.

Here, the fascia is, for example, located at a depth of about 6 mm from the surface of the skin at the shoulder part of the human body, and so that the pressing action and the heating action reach the depth, the shape of the projection part 12 and exothermic performance of the exothermic element 130 are preferably set. Furthermore, the exothermic performance of the exothermic element 130 is preferably set so that, for example, the surface temperature of the skin is equal to or more than 37°C and equal to or less than 44°C, and is more preferably set so that the surface temperature of the skin is equal to or more than 38°C and equal to or less than 42°C.

The number of the projection part 12 included in the projection part sheet 10 is not particularly limited and may be one or plural. Disposition of a plurality of projection parts 12 is not particularly limited but can be, for example, disposition in a staggered lattice shape, a square lattice shape, or the like.

In the case of the present embodiment, for example, as shown in Fig. 1, the projection part sheet 10 has five projection parts 12 disposed in a staggered lattice shape. More specifically, one projection part 12 is disposed at a center portion of the projection part sheet 10, and around this projection part 12, the remaining four projection parts 12 are disposed. These four projection parts 12 are disposed at respective four corner portions of the projection part sheet 10.

A center-to-center distance L of the adjacent projection parts 12 (Fig. 1) is not particularly limited but is preferably equal to or more than the height dimension H1 (Fig. 3) of the projection part 12, and more preferably equal to or more than 1.5 times the height dimension H1. Thereby, a skin of a living body can be sufficiently pressed by the individual projection parts 12.

The heating implement 100 includes an attachment unit 60 for attachment of the heating implement 100 to a living body in a state where the projection part 12 is pressed against the skin.

The attachment unit 60 includes, for example, a pair of attachment band parts 61 each formed in a belt shape slightly elongated in one direction (left-right directions in Figs. 1 and 2) .

As described above, in the case of the present embodiment, the planar shape of the main body sheet 120 is in a rectangular shape. For example, a base end portion 66, which is one end portion of each of the attachment band parts 61 in the longitudinal direction, is fixed along each of a pair of edges of the main body sheet 120 facing each other.

More specifically, the base end portion 66 of each of the pair of attachment band parts 61 is fixed to an outer surface of the second sheet 122 as shown in Fig. 2.

The attachment band part 61 includes a sheet-shaped attachment unit formation sheet 63 and an adhesive layer 64 formed on a surface on one side of a portion on the tip side of the attachment unit formation sheet 63.

The adhesive layer 64 is formed on a surface which is on the skin side of the attachment unit formation sheet 63 when the heating implement 100 is attached to a living body.

In this way, the attachment unit 60 includes an adhesive sheet portion to be adhesively fixed to a skin (for example, a portion of the attachment unit formation sheet 63 at which the adhesive layer 64 is formed).

Thus, the adhesive sheet portion is adhesively fixed to a skin in a state where a tension is applied to the attachment unit 60, and thereby, as shown in Fig. 4, the projection part 12 is pressed against a skin 91, so that the heating implement 100 can be attached to the living body.

A portion of the living body to which the heating implement 100 is attached is not particularly limited. For example, the heating implement 100 can be attached to a body part such as a shoulder or a back, an arm part such as a wrist, a leg part such as a sole, or a head part such as around an eye.

Note that in a pre-use state of the heating implement 100, a release paper 65 covering the adhesive layer 64 is attached to each of the attachment band parts 61.

During the use of the heating implement 100, the release paper 65 is released from each of the attachment band parts 61, and the adhesive layer 64 of each of the attachment band parts 61 is attached to the skin 91, so that the heating implement 100 can be attached to the living body.

Here, in the case of the present embodiment, this attachment unit formation sheet 63 is formed of a material stretchable in the longitudinal direction of the attachment unit formation sheet 63. That is, each of the attachment unit formation sheets 63 is stretchable in an arrow B direction in Fig. 1.

In this way, the attachment unit 60 includes a stretch sheet part having stretchability. In the case of the present embodiment, for example, the attachment unit formation sheet 63 as a whole is the stretch sheet part.

In a state where the attachment band part 61 is stretched in the longitudinal direction of this attachment band part 61, the adhesive layer 64 at a tip portion of the attachment band part 61 is attached to the skin 91, and thereby the projection part 12 can be pressed against the skin 91 with a more sufficient pressing force.

Hereinafter, an example of a material and characteristic of each of the units of the heating implement 100 will be more specifically explained.

As an oxidizable metal in the exothermic material, an oxidizable metal typically used as a material for an exothermic material of this type can be used. As this oxidizable metal, one in a powdery or fibrous form is preferably used in terms of handleability, moldability, and the like.

Examples of the oxidizable metal having a powdery form include iron powder, aluminum powder, zinc powder, manganese powder, magnesium powder, and calcium powder, and of these, iron powder is preferably used in terms of handleability, production cost, and the like.

As the oxidizable metal having a powdery form, in consideration of the improved reaction control, one having a particle size (hereinafter, particle size means a maximum length in a powdery form, or an average particle size measured by a dynamic light scattering method, a laser diffraction method, or the like) of equal to or more than 0.1 µm and equal to or less than 300 µm is preferably used, and one containing equal to or more than 50% by mass of particles having a particle size of equal to or more than 0.1 µm and equal to or less than 150 µm is more preferably used.

Furthermore, examples of the oxidizable metal having a fibrous form include a steel fiber, an aluminum fiber, and a magnesium fiber. Of these, a steel fiber, an aluminum fiber, or the like is preferably used in terms of handleability, production cost, and the like. As the oxidizable metal having a fibrous form, one having a fiber length of equal to or more than 0.1 mm and equal to or less than 50 mm and a thickness of equal to or more than 1 µm and equal to or less than 1000 µm is preferably used in terms of exothermic performance and the like.

The content of the oxidizable metal in the exothermic material is preferably equal to or more than 30% by mass and equal to or less than 80% by mass, and more preferably equal to or more than 40% by mass and equal to or less than 70% by mass.

This content is equal to or more than 30% by mass, and thereby the exothermic temperature of the exothermic element 130 can be sufficiently increased to the extent that a person feels hot by touch with his or her fingertip or the like, which is thus preferable.

This content is equal to or less than 80% by mass, and thereby air permeability of the exothermic material becomes sufficient. As a result, the reaction sufficiently occurs up to a central portion of the exothermic part 133, and the exothermic temperature of the exothermic element 130 can be sufficiently increased. Furthermore, the exothermic element 130 can have a sufficient length of exothermic time, and water supply by a water retention agent can also be made sufficient.

Here, the content of the oxidizable metal in the exothermic material can be measured by an ash test according to JIS P8128 or, in a case where the oxidizable metal is iron, measured by a vibration sample type magnetization measurement test or the like by utilizing the property of causing magnetization when an external magnetic field is applied.

As the water retention agent in the exothermic material, a water retention agent typically used as a material for an exothermic material of this type can be used. This water retention agent serves as a moisture retention agent. Furthermore, this water retention agent may also have a function as a supply agent that retains oxygen supplied to the oxidizable metal and supplies this oxygen to the oxidizable metal.

As this water retention agent, for example, one formed of an inorganic material is preferably used.

As this water retention agent, for example, one formed of a porous material is preferably used.

Examples of the water retention agent include activated carbon (coconut shell carbon, wood charcoal powder, bituminous coal, peat, and lignite), carbon black, acetylene black, graphite, zeolite, perlite, vermiculite, silica, cancrinite, and fluorite, and of these, activated carbon is preferably used in terms of having water retention ability, oxygen supply ability, and catalytic ability.

As this water retention agent, in terms of the capability of forming an effective contact state with the oxidizable metal, one in a powdery form having a particle size of equal to or more than 0.1 µm and equal to or less than 500 µm is preferably used, and one in a powdery form containing equal to or more than 50% by mass of particles having a particle size of equal to or more than 0.1 µm and equal to or less than 200 µm is more preferable.

As this water retention agent, one in a form other than a powdery form as described above can also be used, and, for example, one in a fibrous form such as an activated carbon fiber can also be used.

The content of the water retention agent in the exothermic material is preferably equal to or more than 1% by mass and equal to or less than 50% by mass, and more preferably equal to or more than 2% by mass and equal to or less than 40% by mass.

This content is equal to or more than 1% by mass, and thereby it is possible to sufficiently accumulate in the exothermic material a moisture necessary for maintaining the reaction to the extent that the temperature of the oxidizable metal increases equal to or more than the human body temperature due to the oxidation reaction. Furthermore, the air permeability of the exothermic material is sufficiently ensured, and accordingly oxygen supply to the exothermic material can be sufficiently performed, so that the exothermic efficiency of the exothermic material can be improved.

This content is equal to or less than 50% by mass, and thereby the heat capacity of the exothermic material with respect to the obtained exothermic amount can be suppressed. Thus, the increase in the exothermic temperature becomes large, and the increase in a temperature at which a person can feel warm is obtained.

The exothermic material may include an electrolyte.

As this electrolyte, an electrolyte typically used as a material for an exothermic material of this type can be used.

Examples of this electrolyte include chloride or hydroxide of alkali metal, alkaline earth metal, or heavy metal. Of these, any of various chlorides such as sodium chloride, potassium chloride, calcium chloride, magnesium chloride, and iron chloride (ferrous and ferric) is preferably used in terms of excellent conductivity, chemical stability, and production cost. These electrolytes can also be used alone or in combination of equal to or more than two kinds.

The content of the electrolyte in the exothermic material is preferably, by mass ratio of water in the exothermic material, equal to or more than 0.5% by mass and equal to or less than 24% by mass, and more preferably equal to or more than 1% by mass and equal to or less than 10% by mass.

This content is equal to or more than 0.5% by mass, and thereby the oxidation reaction of the exothermic material can be sufficiently advanced. Furthermore, to ensure an electrolyte necessary for the exothermic function, the water ratio of the exothermic material can also be suppressed. As a result, the increase in the exothermic temperature can be sufficiently ensured.

This content is equal to or less than 24% by mass, and thereby the air permeability of the exothermic material can be improved. Furthermore, to ensure an electrolyte necessary for the exothermic function, the ratio of water in the exothermic material can be maintained at a certain degree of amount. Thereby, sufficient water is supplied to the oxidizable metal or the like, the exothermic performance becomes excellent, and the electrolyte can be uniformly mixed with the exothermic material, which is thus preferable.

Furthermore, a thickener, a flocculant, and moreover other additives may be added to the exothermic material.

The exothermic material includes, for example, an oxidizable metal, a water retention agent, and water. Oxygen is supplied to the oxidizable metal in the exothermic material, and thereby the exothermic material generates heat.

Furthermore, the exothermic material may be one including iron and a carbon component.

The iron referred to here may be at least a part of the above oxidizable metal or may be one different from the above oxidizable metal. The iron referred to here is an oxidizable iron.

Furthermore, the carbon component referred to here may be at least a part of the above water retention agent, or the exothermic material may include a carbon component in addition to the above water retention agent.

In the projection part 12 of the heating implement 100, the exothermic end-point temperature is preferably equal to or more than 35°C and equal to or less than 98°C, more preferably equal to or more than 38°C and equal to or less than 70°C, and still more preferably equal to or more than 42°C and equal to or less than 60°C.

Measurement of the exothermic end-point temperature of the heating implement 100 can be performed by a method equivalent to that of JIS S4100.

In the exothermic element 130, the amount of water vapor generated in 10 minutes per unit weight (1 g) of the exothermic material is preferably equal to or more than 20 mg/g and equal to or less than 250 mg/g, and more preferably equal to or more than 70 mg/g and equal to or less than 180 mg/g.

Here, this amount of water vapor (amount of generated water vapor) is measured, for example, as follows.

A device used for the measurement includes a measurement chamber (volume of 4.2 L) made of aluminum, an inflow path that causes dehumidified air (humidity of less than 2%, flow rate of 2.1 L/min) to flow to a lower portion of the measurement chamber, and an outflow path that causes air to flow out of an upper portion of the measurement chamber. An inlet temperature and humidity meter and an inlet flow meter are attached to the inflow path. On the other hand, an outlet temperature and humidity meter and an outlet flow meter are attached to the outflow path. A thermometer (thermistor) is attached to the inside of the measurement chamber. As the thermometer, one having a temperature resolution of about 0.1°C is used.

The heating implement 100 is taken out of a packaging bag at a temperature of 30°C (30±1°C) in measurement environment and, with the side of the surface on one side 10a of the projection part sheet 10 facing upward, placed on the measurement chamber. Then, the thermometer equipped with a metal ball (mass of 4.5 g) is placed thereon. In this state, the dehumidified air is caused to flow from the lower portion of the measurement chamber, and a difference between absolute humidifies before and after the air flows to the measurement chamber is obtained based on the temperature and the humidity measured by the inlet temperature and humidity meter and the outlet temperature and humidity meter. Furthermore, the amount of water vapor released by the heating implement 100 is calculated based on the flow rates measured by the inlet flow meter and the outlet flow meter. The amount of generated water vapor from the measurement start to elapse of 10 minutes is measured.

Examples of the material of the nonwoven sheet 15 include a synthetic fiber, a natural fiber, and a composite fiber of these, and examples of the manufacturing method include a spunbond method, a needle punching method, a spunlace method, a melt blowing method, a flash spinning method, an air-laid method, and an air-through method.

The nonwoven sheet 15 includes fibers formed of a first resin material, and a binding part formed of a second resin material and binding together the fibers.

The first resin material forming the nonwoven sheet 15 is not particularly limited, but examples thereof include polyethylene, polypropylene, nylon, rayon, polystyrene, acrylic, vinylon, cellulose, aramid, polyvinyl alcohol, polyethylene naphthalate, and polyethylene terephthalate, and of these, polyethylene terephthalate (PET) is preferable.

The second resin material forming the nonwoven sheet 15 is not particularly limited but is preferably a material having a lower melting point than the first resin material forming the nonwoven sheet 15. Examples of the second resin material forming the nonwoven sheet 15 include polyethylene, polypropylene, ethylene vinyl acetate resin, and low melting point PET (copolymerized polyester), and of these, polyethylene or low melting point PET is preferable.

Note that the fiber forming the nonwoven sheet 15 may have a core-sheath structure including a core formed of the first resin material and a sheath formed of the second resin material.

The content of the first resin material in the nonwoven sheet 15 is larger than that of the second resin material in the nonwoven sheet 15.

The content of the first resin material in the nonwoven sheet 15 is preferably equal to or more than 60% by mass and equal to or less than 95% by mass. Furthermore, the content of the second resin material in the nonwoven sheet 15 is equal to or more than 5% by mass and equal to or less than 40% by mass.

The contents of the first and second resin materials in the nonwoven sheet 15 are set in this way, and thereby while air permeability of the nonwoven sheet 15 is sufficiently ensured, rigidity of the nonwoven sheet 15 can be sufficiently ensured.

The thickness of the base part 11 of the projection part sheet 10 is preferably equal to or more than 0.03 mm and equal to or less than 2.6 mm, and particularly preferably equal to or more than 0.08 mm and equal to or less than 1.25 mm. The thickness of the base part 11 is equal to or more than 0.03 mm, and thereby form retainability of the projection part sheet 10 (in particular, form retainability of the projection part 12) and consequently form retainability of the main body 50 are improved. The thickness of the base part 11 is equal to or less than 2.6 mm, and thereby a heat transfer property of the projection part sheet 10 is improved.

In the case of the present embodiment, the degree of moisture permeability of the second sheet 122 is lower than that of the projection part sheet 10.

The degree of moisture permeability of the second sheet 122 and the degree of moisture permeability of the projection part sheet 10 are set in this way, and thereby the generating direction of water vapor associated with the heat generation of the exothermic element 130 can be regulated by the second sheet 122. For example, oxygen supply to the exothermic element 130 is performed from the side of the projection part sheet 10, and generation of the water vapor from the second sheet 122 can be suppressed. Then, the water vapor can be generated mainly from the side of the projection part sheet 10.

The second sheet 122 preferably has a basis weight of equal to or more than 10 g/m² and equal to or less than 200 g/m², and more particularly equal to or more than 20 g/m² and equal to or less than 100 g/m². The basis weight of the second sheet 122 is set within such a range, and thereby the generating direction of the water vapor associated with the heat generation can be regulated by the second sheet 122.

The material of the attachment unit formation sheet 63 is not particularly limited but can be, for example, a nonwoven fabric having stretchability. Examples of the material of this nonwoven fabric include a synthetic fiber, a natural fiber, and a composite fiber of these.

Alternatively, the attachment unit formation sheet 63 is not limited to the nonwoven fabric and may be, for example, a woven fabric including a rubber fiber.

The material of the adhesive layer 64 is not particularly limited, but for example, a rubber type, acrylic type, silicone type, emulsion type, hot melt type, or hydrous gel type adhesive material can be used.

Next, an example of a method for producing the projection part sheet 10 of the heating implement 100 according to the present embodiment will be explained.

First, a nonwoven sheet 18 that serves as an origin of the nonwoven sheet 15 is prepared.

Here, the nonwoven sheet 18 includes, for example, a first fiber formed of a first resin material, and a second fiber formed of a second resin material (in the case of a cotton mix of the first and second fibers). Additionally, the fiber forming the nonwoven sheet 18 may have a core-sheath structure including a core formed of the first resin material and a sheath formed of the second resin material.

Next, heat pressing is performed with respect to the nonwoven sheet 18, and thereby the projection part sheet 10 on which the projection part 12 is formed is molded.

Here, the temperature of heat pressing is set to an intermediate temperature between the melting point of the first resin material and that of the second resin material. That is, the temperature of heat pressing is set to a temperature less than the melting point of the first resin material and equal to or more than that of the second resin material.

Thus, while the second resin material melts, the first resin material can be prevented from melting, and accordingly the fibers (these fibers may be portions of the core of the core-sheath structure) formed of the first resin material are bound together via the melted second resin material. That is, the melted second resin material forms the binding part binding together the fibers formed of the first resin material.

As a result, while the air permeability of the projection part sheet 10 is ensured, the rigidity of the projection part sheet 10 can be sufficiently ensured. That is, air permeability and rigidity of the base part 11 can be sufficiently ensured, and the projection part 12 can also have a sufficient rigidity as a whole while the air permeability is ensured from a base end of this projection part 12 to a tip thereof.

Here, the temperature of heat pressing is preferably set to be as low as possible within a range in which the second resin material can sufficiently melt (for example, a temperature obtained by adding equal to or less than 30°C to the melting point of the second resin material, and preferably a temperature obtained by adding equal to or less than 20°C to the melting point of the second resin material). Thereby, the nonwoven sheet 15 after heat pressing can be made to have a texture of nonwoven fabric, and a feel of the main body 50 on the skin is improved.

Here, for example, as shown in Fig. 5A, the projection part 12 can be formed on the projection part sheet 10 by using a first mold 70 and a second mold 80 disposed to face each other.

The first mold 70 includes a flat surface 71 facing the second mold 80, and a plurality of projection parts 72 projecting from the flat surface 71 toward the side of the second mold 80.

The second mold 80 includes a flat surface 81 facing the first mold 70, and a plurality of concave parts 82 each formed at a portion facing each of the projection parts 72 in the flat surface 81.

As shown in Fig. 5B, the first mold 70 and the second mold 80 are brought close to each other to press the projection part sheet 10 in the thickness direction, and the projection part sheet 10 is heated by the first mold 70 and the second mold 80, thereby forming the plurality of projection parts 12 on the projection part sheet 10. In the projection part sheet 10, a portion corresponding to the flat surface 71 of the first mold 70 and the flat surface 81 of the second mold 80 is the base part 11, and a portion corresponding to the projection part 72 of the first mold 70 and the concave part 82 of the second mold 80 is the projection part 12.

Here, an example of preferable molding conditions of the projection part sheet 10 will be explained.

The pressing temperature (molding temperature) is preferably equal to or more than 90°C and equal to or less than 220°C, and more preferably equal to or more than 100°C and equal to or less than 200°C.

The basis weight of the nonwoven sheet 15 is preferably equal to or more than 15 g/m² and equal to or less than 500 g/m², more preferably equal to or more than 30 g/m² and equal to or less than 350 g/m², and still more preferably equal to or more than 100 g/m² and equal to or less than 250 g/m². The basis weight of the nonwoven sheet 15 is equal to or more than 15 g/m², and thereby a sufficient strength of the projection part sheet 10 can be ensured, and the temperature of the exothermic element 130 can be suitably moderated and transmitted to a skin. The basis weight of the nonwoven sheet 15 is equal to or less than 500 g/m², and thereby the temperature of the exothermic element 130 can be effectively transmitted to a skin via the projection part sheet 10.

The pressing time is preferably equal to or more than 0.5 second and equal to or less than 200 seconds, and more preferably equal to or more than 1 second and equal to or less than 100 seconds.

Next, modified examples of the disposition of the projection part 12, the shape of the projection part 12, and the like will be explained with reference to Figs. 7A to 7D.

### <Modified Example 1>

Figs. 7A and 7B are views for explaining Modified Example 1 of the planar shape of the projection part sheet 10, the disposition of the projection part 12, and the shape of the projection part 12, and of these, Fig. 7A is a plan view, and Fig. 7B is a cross-sectional view taken along an A-A line of Fig. 7A.

In the case of the present modified example, the projection part 12 is formed in a truncated circular cone shape. That is, an apex portion of the projection part 12 is formed flat.

Furthermore, the projection parts 12 are disposed in a staggered lattice shape, and the projection part sheet 10 is provided with, for example, ten projection parts 12 in total in three horizontal rows.

The planar shape of the projection part sheet 10 is formed in, for example, a hexagonal shape.

### <Modified Example 2>

Figs. 7C and 7D are views for explaining Modified Example 2 of the planar shape of the projection part sheet 10, the disposition of the projection part 12, and the shape of the projection part 12, and of these, Fig. 7C is a plan view, and Fig. 7D is a cross-sectional view taken along an A-A line of Fig. 7C.

In the case of the present modified example, the projection part sheet 10 has a plurality of types of projection parts 12 different in shape from each other.

Furthermore, in the case of the present modified example, the projection part sheet 10 has a plurality of types of projection parts 12 different in dimensions from each other.

More specifically, in the case of the present modified example, the planar shape of the projection part sheet 10 is, for example, the same as that of Modified Example 1. Then, one horizontally elongated elliptical projection part 12 (hereinafter, a first projection part 12a) is disposed at the center portion of the projection part sheet 10, and around the first projection part 12a, a plurality of (for example, eight) projection parts 12 (hereinafter, second projection parts 12b) is annularly disposed at an equal interval.

An apex portion of the first projection part 12a has a horizontally elongated ridge (see Fig. 7D).

A disposition region of the first projection part 12a of the projection part sheet 10 of the present modified example corresponds to those of two projection parts 12 at the center portion of the projection part sheet 10 of Modified Example 1.

That is, dimensions of the first projection part 12a and those of the second projection part 12b are different from each other, and for example, when viewed in the direction perpendicular to the surface of the projection part sheet 10, outer dimensions of the first projection part 12a are larger than those of the second projection part 12b.

The planar shape of the first projection part 12a is, for example, elliptical. On the other hand, the planar shape of the second projection part 12b is, for example, circular. That is, the first projection part 12a and the second projection part 12b are different in shape from each other.

### [Second embodiment]

Next, the second embodiment will be explained with reference to Figs. 8 to 9B.

The heating implement 100 according to the present embodiment differs from the heating implement 100 according to the above first embodiment in the configuration of the projection part sheet 10 and is otherwise configured similarly to the heating implement 100 according to the above first embodiment.

In the above first embodiment, an example in which the projection part sheet 10 is formed of one nonwoven sheet 15 is explained.

In contrast, in the present embodiment, the projection part sheet 10 includes the nonwoven sheet 15 (first nonwoven sheet) forming one outermost layer of this projection part sheet 10, a nonwoven sheet 17 (second nonwoven sheet) forming the other outermost layer of this projection part sheet 10, and an air permeable sheet 16 forming an intermediate layer located between the first nonwoven sheet and the second nonwoven sheet.

More specifically, in the case of the present embodiment, the projection part sheet 10 has, for example, as shown in Fig. 8, a three-layer structure of the nonwoven sheet 15, the air permeable sheet 16, and the nonwoven sheet 17.

Note that the present invention is not limited to this example, and the projection part sheet 10 may include a layer other than the three layers of the nonwoven sheet 15, the air permeable sheet 16, and the nonwoven sheet 17. As an example, the projection part sheet 10 may include two layers of the air permeable sheet 16 between the nonwoven sheet 15 and the nonwoven sheet 17 and further include a third nonwoven sheet between these two layers of the air permeable sheet 16, having a five-layer structure in total.

As explained in the above first embodiment, the nonwoven sheet 15 includes the fibers formed of the first resin material, and the binding part formed of the second resin material and binding together the fibers. Furthermore, similarly to the nonwoven sheet 15, the nonwoven sheet 17 also includes fibers formed of a first resin material, and a binding part formed of a second resin material and binding together the fibers.

That is, each of the first nonwoven sheet and the second nonwoven sheet includes the fibers formed of the first resin material, and the binding part formed of the second resin material and binding together the fibers.

Alternatively, the first resin material forming the nonwoven sheet 15 and the first resin material forming the nonwoven sheet 17 may be the same material or may be materials different from each other.

Furthermore, the second resin material forming the nonwoven sheet 15 and the second resin material forming the nonwoven sheet 17 may be the same material or may be materials different from each other.

In the case of the present embodiment, for example, the nonwoven sheet 15 and the nonwoven sheet 17 are formed of the same material, the first resin material forming the nonwoven sheet 15 and the first resin material forming the nonwoven sheet 17 are the same material, and the second resin material forming the nonwoven sheet 15 and the second resin material forming the nonwoven sheet 17 are the same material.

Furthermore, the basis weight of the nonwoven sheet 17 can be appropriately set similarly to that of the nonwoven sheet 15.

Note that in the case of the present embodiment, the fiber forming the nonwoven sheet 15 may also have a core-sheath structure including a core formed of the first resin material and a sheath formed of the second resin material.

Furthermore, the fiber forming the nonwoven sheet 17 may similarly have a core-sheath structure including a core formed of the first resin material and a sheath formed of the second resin material.

The degree of air permeability of the nonwoven sheet 17 is similar to that of the nonwoven sheet 15, which is preferably equal to or more than 1 second/100 ml, and more preferably equal to or more than 3 seconds/100 ml. Furthermore, it is preferably equal to or less than 20000 seconds/100 ml, and more preferably equal to or less than 10000 seconds/100 ml.

The air permeable sheet 16 includes a third resin material having a higher melting point than the second resin material.

Air permeability of the air permeable sheet 16 is not particularly limited, but for example, the degree of moisture permeability of the air permeable sheet 16 is preferably equal to or more than 100 g/(m²·24 h) and equal to or less than 13000 g/(m²·24 h), and particularly preferably equal to or more than 200 g/(m²·24 h) and equal to or less than 8000 g/(m²·24 h). The degree of moisture permeability of the air permeable sheet 16 is set within such a range, and thereby when the heating implement 100 is taken out of a packaging material, oxygen is quickly supplied to the exothermic element 130 through the projection part sheet 10, so that heat and water vapor can be quickly generated from this exothermic element 130, and duration of the heat generation can be sufficiently prolonged. Measurement of the degree of moisture permeability of the air permeable sheet 16 can be performed by, for example, a JIS (Z0208) CaCl₂ method, and the measurement conditions can be 40°C and RHM of 90%.

The air permeable sheet 16 may have air permeability over an entire surface thereof or may partially have air permeability.

The air permeable sheet 16 preferably has a basis weight of equal to or more than 10 g/m² and equal to or less than 200 g/m², and particularly preferably equal to or more than 20 g/m² and equal to or less than 100 g/m². The basis weight of the air permeable sheet 16 is set within such a range, and thereby when the heating implement 100 is taken out of the packaging material, heat and water vapor can be quickly generated, and duration of the heat generation can be sufficiently prolonged.

Examples of the air permeable sheet 16 include one in which an air hole is mechanically formed on a sheet formed of a resin such as polyolefin such as polyethylene or polypropylene, polyester, polyamide, polyurethane, polystyrene, or polyethylene vinyl acetate copolymer, one in which a mixed sheet of these resins and an inorganic filler is interfacially peeled by stretching and provided with a fine air hole, one in which a fine air hole is formed by using interfacially peeling of the crystal structure, and one in which fine air holes are communicated with each other by using open cells by foam molding. Furthermore, examples of the air permeable sheet 16 also include synthetic pulp such as polyolefin, wood pulp, a semi-synthetic fiber such as rayon and acetate, a nonwoven fabric formed from vinylon fiber, polyester fiber or the like, a woven fabric, synthetic paper, and paper.

The air permeable sheet 16 can also be used by stacking a plurality thereof.

More specifically, as the air permeable sheet 16, one can be preferably used in which a mixed sheet of polypropylene and calcium carbonate is interfacially peeled by stretching, and thereby a fine air hole is formed on this mixed sheet.

In the present embodiment, assuming that the air permeable sheet 16 is configured by stretching the mixed sheet of polypropylene and calcium carbonate, the following explanation will be made.

Next, an example of a method for producing the projection part sheet 10 of the heating implement according to the present embodiment will be explained.

First, a nonwoven sheet 18 that serves as an origin of the nonwoven sheet 15, the air permeable sheet 16, and a nonwoven sheet 19 that serves as an origin of the nonwoven sheet 17 are prepared, and these three sheets are laminated so that the nonwoven sheet 18, the air permeable sheet 16, and the nonwoven sheet 19 are stacked in this order.

As described above, the nonwoven sheet 18 includes, for example, a first fiber formed of a first resin material, and a second fiber formed of a second resin material. Additionally, the fiber forming the nonwoven sheet 18 may have a core-sheath structure including a core formed of the first resin material and a sheath formed of the second resin material.

The nonwoven sheet 19 is, for example, similar to the nonwoven sheet 18.

Next, heat pressing is performed with respect to a laminate of these three sheets (nonwoven sheet 18, air permeable sheet 16, and nonwoven sheet 19), and thereby the projection part sheet 10 on which the projection part 12 is formed is molded (see Figs. 9A and 9B) .

In the present embodiment, the temperature of heat pressing is also set to an intermediate temperature between the melting point of the first resin material and that of the second resin material. That is, the temperature of heat pressing is set to a temperature less than the melting point of the first resin material and equal to or more than that of the second resin material.

Thus, while the second resin material melts, the first resin material can be prevented from melting, and accordingly the fibers (these fibers may be portions of the core of the core-sheath structure) formed of the first resin material are bound together via the melted second resin material. That is, the melted second resin material forms the binding part binding together the fibers formed of the first resin material.

Thus, while air permeabilities of the nonwoven sheet 15 and the nonwoven sheet 17 are ensured, rigidities of the nonwoven sheet 15 and the nonwoven sheet 17 can be sufficiently ensured, and accordingly the air permeability and rigidity of the projection part sheet 10 can be sufficiently ensured. That is, the air permeability and rigidity of the base part 11 can be sufficiently ensured, and the projection part 12 can also have a sufficient rigidity as a whole while the air permeability is ensured from a base end of this projection part 12 to a tip thereof.

In the case of the present embodiment, the temperature of heat pressing is also preferably set to be as low as possible within a range in which the second resin material can sufficiently melt (for example, a temperature obtained by adding equal to or less than 30°C to the melting point of the second resin material, and preferably a temperature obtained by adding equal to or less than 10°C to the melting point of the second resin material). Thereby, the nonwoven sheet 15 and the nonwoven sheet 17 after heat pressing can be made to have a texture of nonwoven fabric. In particular, the nonwoven sheet 17 located on the side of the outer surface of the main body 50 has a texture of nonwoven fabric, and thereby a feel of the main body 50 is improved.

In the case of the present embodiment, the temperature of heat pressing is preferably set to a temperature lower than the melting point of the third resin material included in the air permeable sheet 16 and lower than the stretching temperature of the air permeable sheet 16. Thus, the air hole of the air permeable sheet 16 can be maintained even after heat pressing, and air permeability of this air permeable sheet 16 can be ensured.

Thus, in the present embodiment, in a state where the nonwoven sheet 18 and the nonwoven sheet 19 are respectively stacked on both surfaces of the air permeable sheet 16 including the third resin material, these three sheets (nonwoven sheet 18, air permeable sheet 16, and nonwoven sheet 19) are heat pressed, and thereby the projection part 12 is formed.

Thus, at the time of heat pressing, the nonwoven sheets 18 and 19 can protect the respective both surfaces of the air permeable sheet 16. Thus, even in a case where the air permeable sheet 16 subjected to heat pressing and included in the projection part sheet 10 includes, for example, the third resin material having a high crystallinity such as polypropylene, while rupture of the air permeable sheet 16 is prevented, the projection part 12 can be formed on the projection part sheet 10. Accordingly, the projection part sheet 10 can be made to have a uniform air permeability over an entire surface thereof.

Furthermore, the projection part sheet 10 after heat pressing has a laminated structure in which the nonwoven sheets 15 and 17 are disposed on the respective both surfaces of the air permeable sheet 16. Thus, the rigidity of the projection part sheet 10 can be more easily ensured, and in particular, in the projection part 12, the rigidity can also be ensured in an improved manner.

Note that in the projection part sheet 10 after heat pressing, the second resin material of the nonwoven sheet 15 and the second resin material of the nonwoven sheet 17 may or may not be bound to the air permeable sheet 16.

In the case of the present embodiment, the second resin material of the nonwoven sheet 15 and the second resin material of the nonwoven sheet 17 are not bound to the air permeable sheet 16, and thus the air permeability of the air permeable sheet 16 can be maintained in an improved manner.

Note that although in the second embodiment, an example is mainly explained in which one configured by stretching the mixed sheet of polypropylene and calcium carbonate is used as the air permeable sheet 16, the present invention is not limited to this example. For example, the air permeable sheet 16 may be produced by forming a plurality of pores on a resin sheet formed of the third resin material. That is, the air permeable sheet 16 is, for example, a resin sheet formed of the third resin material and has a plurality of pores penetrating front and rear sides of this air permeable sheet 16.

Note that before the nonwoven sheet 18 and the nonwoven sheet 19 are stacked on the respective both sides of the air permeable sheet 16 at the time of the above described heat pressing, it is also preferable to attach a nonwoven fabric to the air permeable sheet 16 in advance to reinforce the air permeable sheet 16.

### [Third embodiment]

Next, the third embodiment will be explained with reference to Figs. 10A and 10B.

A heating implement (not entirely shown in the drawings) according to the present embodiment differs from the above first or second embodiment in the shape of the projection part 12 of the projection part sheet 10 and is otherwise configured similarly to the heating implement according to the above first or second embodiment.

In the case of the present embodiment, the projection part 12 has a multi-stage structure including a first stage portion 212a and a second stage portion 212b disposed on the tip side in the projecting direction of this projection part 12 relative to the first stage portion 212a and having smaller dimensions than the first stage portion 212a when this projection part 12 is viewed in the projecting direction of this projection part 12.

More specifically, in the case of the present embodiment, the projection part 12 has a two-stage structure of the first stage portion 212a and the second stage portion 212b. Note that the present invention is not limited to this example, and the projection part 12 may be formed in a stage structure having equal to or more than three stages.

More specifically, the first stage portion 212a has, for example, a hemispherical shape (dome shape). Furthermore, the second stage portion 212b has, for example, a circular cone shape. Note that an apex portion of the second stage portion 212b is rounded. Furthermore, the first stage portion 212a and the second stage portion 212b are mutually concentrically disposed.

The projection part 12 has the multi-stage structure, and thereby, for example, in a state where a part of the stage (for example, the first stage portion 212a) is crushed, a comfortable pressure point pressing can be performed by the projection part 12 (mainly, the second stage portion 212b). Furthermore, when this projection part 12 is viewed in the projecting direction of the projection part 12, the dimensions of the second stage portion 212b are smaller than those of the first stage portion 212a, and accordingly a skin can be locally pressed by the second stage portion 212b, enabling to provide a more comfortable pressure point pressing.

### [Fourth embodiment]

Next, the fourth embodiment will be explained with reference to Figs. 11A to 11D.

A heating implement (not entirely shown in the drawings) according to the present embodiment differs from the above first or second embodiment in the shape of the projection part 12 of the projection part sheet 10 and is otherwise configured similarly to the heating implement according to the above first or second embodiment.

In the case of the present embodiment, the projection part 12 has an annular concave portion 212c disposed between the first stage portion 212a and the second stage portion 212b. Furthermore, an apex portion of the first stage portion 212a is, for example, an annular flat portion 212d. The first stage portion 212a, the concave portion 212c, and the second stage portion 212b are mutually concentrically disposed.

In the case of the present embodiment, similarly to the case of the above third embodiment, for example, in a state where the first stage portion 212a is crushed, a comfortable pressure point pressing can be performed by the projection part 12 (mainly, the second stage portion 212b).

Furthermore, in the case of the present embodiment, when a skin is pressed by the second stage portion 212b for pressure point pressing, the concave portion 212c elastically deforms, and thereby pressure point pressing can be made by the second stage portion 212b with a sufficient elastic force.

The present invention is not limited to each of the above embodiments and modified examples and also includes aspects of various modifications and improvements and the like as long as the object of the present invention is achieved.

For example, the exothermic material may include an oxidizable metal, a water retention agent, water, and a water absorbing polymer.

The exothermic material includes a water absorbing polymer, and thereby excessive water in the exothermic material can be absorbed by the water absorbing polymer. Accordingly, once the heating implement 100 is taken out of the packaging material, the exothermic element 130 can quickly generate heat.

The content of the water absorbing polymer in the exothermic material is preferably equal to or more than 1% by mass and equal to or less than 12% by mass, and more preferably equal to or more than 2% by mass and equal to or less than 8% by mass. The content of the water absorbing polymer in the exothermic material is equal to or more than 1% by mass, and thereby water absorption can be sufficiently performed by the water absorbing polymer. Furthermore, the content of the water absorbing polymer in the exothermic material is equal to or less than 12% by mass, and thereby the content of, in the exothermic material, the oxidizable metal that contributes to heat generation can be sufficiently ensured.

Furthermore, although in the above embodiment, an example in which the attachment unit 60 includes the pair of adhesive attachment band parts 61 is explained, the present invention is not limited to this example. For example, the main body 50 may be wound around a leg, an arm, or the like by using a belt-shaped body such as a bandage, and the projection part 12 may be pressed against the skin.

Furthermore, the attachment unit 60 may have a form as in an eye mask including a pair of ear hooks that can be hung on user's ears. That is, the attachment unit 60 may include a pair of ear hooks instead of the pair of attachment band parts 61.

Furthermore, the attachment unit 60 may be, for example, a U-shaped plate member integrally molded of an elastically deformable resin material. That is, the attachment unit 60 may include a pair of facing parts disposed to face each other, and a connecting part connecting these facing parts to each other.

In this case, in a state where the main body 50 is put on an inner surface of one of the facing parts by attachment or the like, a facing distance between the pair of facing parts is widened, and furthermore, in that state, a palm or the like is inserted into the facing distance between the pair of facing parts, thereby releasing the force that widens the facing distance between the pair of facing parts. Thus, the attachment unit 60 elastically returns, and accordingly, for example, the projection part 12 is pressed against a skin at a portion between the thumb and the forefinger in the palm, and a pressure point or the like located at this portion can be pressed by the projection part 12.

### Examples

Hereinafter, examples and comparative examples will be explained.

In each of Examples 1 to 10, a projection part sheet similar to that of the above first embodiment was produced. That is, in each of Examples 1 to 10, a nonwoven sheet including fibers formed of a first resin material, and a binding part formed of a second resin material having a lower melting point than the first resin material and binding together the fibers, was press molded, and thereby the projection part sheet was produced. In each of Examples 1 to 10, the first resin material is PET, and the second resin material is low melting point PET. In each of Examples 1 to 10, molding conditions of the projection part sheet were made different. Note that in each of Examples 1 to 9, the height of a projection part was about 6 mm, and in Example 10, the height of a projection part was 6.5 mm.

In Examples 11 and 12, a projection part sheet similar to that of the fourth embodiment (Figs. 11A to 11D) was produced. In Examples 11 and 12, a nonwoven sheet similar to those of Examples 1 to 10 was also used, and the height of a projection part was 6.0 mm.

In Examples 13 and 14, a projection part sheet was produced in which the shape of a projection part is a shape resembling that of the third embodiment (Figs. 10A and 10B), and the planar shape of the first stage portion 212a is in a rounded star shape (a flower-like shape having five petals). In Examples 13 and 14, a nonwoven sheet similar to those of Examples 1 to 10 was also used, and the height of the projection part was 6.0 mm.

In Example 15, a projection part sheet in which the planar shape of a projection part is elliptical was produced. In Example 15, a nonwoven sheet similar to those of Examples 1 to 10 was also used, and the height of the projection part was 10.0 mm.

Fig. 25 shows, for example, molding conditions of the projection part sheet of each of the examples.

The molding temperature (pressing temperature) was 110°C in Examples 1, 5, and 6, 160°C in Examples 2, 4, and 7, and 185°C in Examples 3, 8, and 9.

The basis weight of the nonwoven sheet was 140 g/m² in Examples 1, 4, and 8, 190 g/m² in Examples 2, 5, and 9, and 330 g/m² in Examples 3, 6, and 7.

The pressing time was 10 seconds in Examples 1, 2, and 3, 30 seconds in Examples 5, 7, and 8, and 50 seconds in Examples 4, 6, and 9.

Furthermore, although not shown in the drawings, in Example 10, the molding temperature was 160°C, the basis weight of the nonwoven sheet was 190 g/m², and the pressing time was 10 seconds. In Example 11, the molding temperature was 110°C, the basis weight of the nonwoven sheet was 210 g/m², and the pressing time was 50 seconds. Example 12 differs from Example 11 only in the basis weight of the nonwoven sheet, and this basis weight was 150 g/m². In Example 13, the molding temperature was 110°C, the basis weight of the nonwoven sheet was 210 g/m², and the pressing time was 50 seconds. Example 14 differs from Example 13 only in the basis weight of the nonwoven sheet, and this basis weight was 150 g/m². In Example 15, the molding temperature was 120°C, the basis weight of the nonwoven sheet was 210 g/m², and the pressing time was 20 seconds.

Furthermore, in each of the comparative examples, a projection part sheet in a shape similar to that of each of the examples was produced by using a nonwoven sheet formed of a fiber of amorphous PET. In each of the comparative examples, molding conditions of the projection part sheet were made different.

The molding temperature (pressing temperature) was 100°C in Comparative Example 1, 120°C in Comparative Example 2, and 110°C in Comparative Example 3.

The basis weight of the nonwoven sheet of amorphous PET was 50 g/m² in Comparative Example 1, 150 g/m² in Comparative Example 2, and 250 g/m² in Comparative Example 3.

The pressing time was 5 seconds in Comparative Example 1, also 5 seconds in Comparative Example 2, and also 5 seconds in Comparative Example 3.

In each of the examples and comparative examples, for one projection part, a profile showing a relationship between a magnitude of a load when this projection part was pressed in the direction opposite to the projecting direction of this projection part, and an amount of crush of the projection part, was obtained.

Specifically, measurement was performed according to JIS K7181 (Plastics-Determination of compressive properties) as follows.

As a measurement device, a Tensilon UCT-100W (manufactured by ORIENTEC CORPORATION) was used. The projection part sheet was placed on a horizontal support table, and while a load was applied to one projection part in the direction opposite to the projecting direction of this projection part, the magnitude of the load and the amount of crush of the projection part were measured. The speed of pressing the projection part was 3 mm/min. As a load cell for measuring the magnitude of the load, one having a capacity of 250 N was used.

In each of the examples and comparative examples, from samples produced under the same conditions, three portions including the projection part were cut out to produce three samples, and each sample was measured. That is, in each of the examples and comparative examples, the number of measurements was set to three times, and the profile was obtained based on the average value of the three measurement results.

The curve L51 shown in Fig. 12 shows the profile of the projection part of the projection part sheet of Example 10. Fig. 13 shows plot points forming the profile of Fig. 12. Fig. 14 is a view showing plot points of a slope of the profile of the relationship between the load and the amount of crush on the projection part of Example 10.

In Fig. 13, the plot interval is 0.05 mm, and the plot interval (an interval between sampling values) of the above profile on the second axis is 1/130 of the height dimension of the projection part. In FIG. 13, ninety-eight plot points in total in which the load is in the range of equal to or less than 100 N are plotted.

As described above, in the profiles of Figs. 12 and 13, the boundary point between the first region R1 and the second region R2 is the upper yield point, that is, the plot point P1. Furthermore, the boundary point between the second region R2 and the third region R3 is the intersection point P3.

The curve L51 shown in Fig. 15 shows the profile of the projection part of the projection part sheet of Example 11. Fig. 16 shows plot points forming the profile of Fig. 15. A curve L71 shown in Fig. 17 shows plot points of a slope of the profile of the relationship between the load and the amount of crush on the projection part of Example 11, and a curve L72 shown in Fig. 17 shows, on the projection part of Example 11, a correlation coefficient between each approximate straight line obtained by the above described least squares method and five sampling values included in the unit sample group corresponding to this approximate straight line.

In Fig. 16, the plot interval is 0.05 mm, and the plot interval (an interval between sampling values) of the above profile on the second axis is 1/130 of the height dimension of the projection part. In FIG. 17, ninety-nine plot points in total are plotted in the range in which the load is equal to or less than 100 N.

In the profiles of Figs. 15 and 16, the boundary point between the first region R1 and the second region R2 is the upper yield point, that is, the plot point P1. Furthermore, the boundary point between the second region R2 and the third region R3 is the intersection point P3.

Furthermore, in the other examples and comparative examples, by means of the above described method, the upper yield point or the 1% yield strength point is also set to the boundary point between the first region R1 and the second region R2, and the intersection point between the 1% reverse offset straight line (see the 1% reverse offset straight line L44 of Fig. 13) and the profile is set to the boundary point between the second region R2 and the third region R3. Note that in Figs. 18 to 20, for example, the ranges of the first region R1, the second region R2, and the third region R3 and the slopes of the first polygonal line portion R31, the second polygonal line portion R32, and the third polygonal line portion R33 are conceptually shown and accordingly not necessarily accurately shown in the drawings.

Fig. 18 shows the profile of the projection parts of the projection part sheets of Examples 1, 2, and 3. In Fig. 18, the vertical axis (first axis) shows the magnitude of the load on the projection part, and the horizontal axis (second axis) shows the amount of crush of the projection part. The profile of Example 1 is a curve L11, the profile of Example 2 is a curve L12, and the profile of Example 3 is a curve L13.

Fig. 19 is a view of Fig. 18 enlarged in the vertical axis direction. However, the profile of the third example (curve L13) is not shown in Fig. 19.

Fig. 20 is a view of Fig. 19 further enlarged in the vertical axis direction. However, the profile of the second example (curve L12) is not shown in Fig. 20.

Note that in the profile of Example 1 (curve L11), as shown in Fig. 19, in the third region R3, the range having the load of about 55 N to 100 N (broken line portion) was estimated from the range having the load of less than 55 N.

Fig. 21 shows the profile of the projection parts of the projection part sheets of Comparative Examples 1, 2, and 3. In Fig. 21, the vertical axis (first axis) shows the magnitude of the load on the projection part, and the horizontal axis (second axis) shows the amount of crush of the projection part. The profile of Comparative Example 1 is a curve L21, the profile of Comparative Example 2 is a curve L22, and the profile of Comparative Example 3 is a curve L23.

Fig. 22 is a view of Fig. 21 enlarged in the vertical axis direction. However, the profile of the third comparative example (curve L23) is not shown in Fig. 22.

Fig. 23 is a view of Fig. 22 further enlarged in the vertical axis direction. However, the profile of the second comparative example (curve 21) is not shown in Fig. 23.

Note that in the profile of Comparative Example 1 (curve L21), as shown in Figs. 21 to 23, in the third region R3, the range having the load of about 1.8 N to 100 N (broken line portion) was estimated from the range having the load of less than 1.8 N. Similarly, in the profile of Comparative Example 2 (curve L22), as shown in Figs. 21 and 22, in the third region R3, the range having the load of about 47 N to 100 N (broken line portion) was estimated from the range having the load of less than 47 N.

Fig. 24 shows the profile of the projection parts of the projection part sheets of Examples 4, 5, and 6. In Fig. 24, the vertical axis (first axis) shows the magnitude of the load on the projection part, and the horizontal axis (second axis) shows the amount of crush of the projection part.

As shown in any of Figs. 12, 15, and 18 to 20, in any of Examples 10, 11, and 1 to 3, the profile (curves L11, L12, and L13) of the relationship between the load and the amount of crush includes the first region R1 in which the amount of crush increases as the load increases, and the second region R2 located on the side (on the right side in each of Figs. 12, 15, and 18 to 20) in which the value on the second axis (horizontal axis) is larger than that in the first region R1 and having a larger increase rate of the amount of crush associated with increase in the load than the first region R1, and the range of the second region R2 (length L2) is wider than that of the first region R1 (length L1) in the direction of the second axis (left-right direction in each of Figs. 12, 15, and 18 to 20).

Thus, the reaction force from the projection part can be prevented from becoming excessive, and accordingly it is considered that a skin of a living body such as a human body can be pressed by the projection part in a more comfortable manner.

Furthermore, in any of Examples 10, 11, and 1 to 3, the above profile further includes the third region R3 located on the side in which the value on the second axis is larger than that in the second region R2 and having a smaller increase rate of the amount of crush associated with increase in the load than the second region R2.

Furthermore, in any of Examples 10, 11, and 1 to 3, when the above profile is approximated by three polygonal lines continuous with each other, a region corresponding to the first polygonal line portion R31 is the first region R1, a region corresponding to the second polygonal line portion R32 adjacent to the first polygonal line portion R31 is the second region R2, and a region corresponding to the third polygonal line portion R33 adjacent to the second polygonal line portion R32 is the third region R3.

Furthermore, in any of Examples 1 to 3 and 11, the load is 0 at one end of the first region R1. Although a plot point in which the load is 0 is not shown in Fig. 13 of Example 10, the load is also 0 at one end of the first region R1 in Example 10.

Furthermore, in any of Examples 10, 11, and 1 to 3, the third region R3 is in the range in which the above load is equal to or less than 100 N.

Furthermore, in any of Examples 10, 11, and 1 to 3, the second region R2 includes a point in which the above amount of crush is 1/4 of the height of the projection part (a point in which the amount of crush is 1.625 mm in Example 10, and a point in which the amount of crush is 1.5 mm in Examples 1 to 3).

Furthermore, in any of Examples 10, 11, and 1 to 3, the minimum value of the above load in the second region R2 is equal to or more than 0.2 N.

Furthermore, in any of Examples 10, 11, and 1 to 3, it was confirmed that the projection part has air permeability.

Furthermore, in Examples 10 and 11, assuming that the load at the boundary point between the first region R1 and the second region R2 (plot point P1) is set to F1, and the load at the second region division point P4 is set to F2, 0.8 < (F2/F1) ≤ 2 is satisfied. Furthermore, in Example 10, 1 < (F2/F1) ≤ 1.5 is satisfied.

Furthermore, in Examples 10 and 11, in the direction of the second axis, the range of the second region former half part R21 (length L201) is wider than that of the second region latter half part R22 (length L202). Furthermore, in Examples 10 and 11, the length L201 is equal to or more than twice the length L202. In particular, in Example 11, the length L201 is equal to or more than three times the length L202.

On the other hand, as shown in any of Figs. 21 to 23, in any of Comparative Examples 1 to 3, unlike the present invention, in the direction of the second axis (left-right direction in each of Figs. 21 to 23), the range of the first region R1 (length L1) is wider than that of the second region R2 (length L2).

Furthermore, in any of Comparative Examples 1 to 3, the second region does not include a point in which the above amount of crush is 1/4 of the height of the projection part (a point in which the amount of crush is 1.5 mm).

Furthermore, in Examples 4, 5, and 6 shown in Fig. 24, the profile of the relationship between the load and the amount of crush also includes the first region in which the amount of crush increases as the load increases, and the second region located on the side (on the right side in Fig. 24) in which the value on the second axis (horizontal axis) is larger than that in the first region and having a larger increase rate of the amount of crush associated with increase in the load than the first region, and the range of the second region R2 (length) is wider than that of the first region (length) in the direction of the second axis (left-right direction in Fig. 24).

Furthermore, although not shown in the drawings, it was confirmed that the same applies to any of Examples 7 to 9 and 12 to 15.

Furthermore, although not shown in the drawings, in any of Examples 4 to 9 and 12 to 15, it was confirmed that the above profile further includes the third region located on the side in which the value on the second axis is larger than that in the second region and having a smaller increase rate of the amount of crush associated with increase in the load than the second region.

Furthermore, in any of Examples 4 to 9 and 12 to 15, it was confirmed that when the above profile is approximated by three polygonal lines continuous with each other, a region corresponding to the first polygonal line portion is the first region, a region corresponding to the second polygonal line portion adjacent to the first polygonal line portion is the second region, and a region corresponding to the third polygonal line portion adjacent to the second polygonal line portion is the third region.

Furthermore, in any of Examples 4 to 9 and 12 to 15, the load was 0 at one end of the first region, the third region was in the range having the above load of equal to or less than 100 N, the second region included a point in which the above amount of crush was 1/4 of the height of the projection part (a point in which the amount of crush was 1.5 mm), the minimum value of the above load in the second region R2 was equal to or more than 0.2 N, and the projection part had air permeability.

Furthermore, in any of Examples 12 to 15, it was confirmed that 0.8 < (F2/F1) ≤ 3 is satisfied.

Note that as shown in Fig. 25, it is considered that the pressure applied to the projection part when the above amount of crush is 1/4 of the height of the projection part increases as the molding temperature increases and increases as the basis weight of the nonwoven sheet increases.

This application claims priority based on Japanese Patent Application No. 2018-060660 filed on March 27, 2018, and the entire disclosure thereof is incorporated herein.

### Reference Signs List

- 10: Sheet
- 10a: Surface on one side
- 10b: Surface on the other side
- 11: Base part
- 12: Projection part
- 12a: First projection part
- 12b: Second projection part
- 13: Cavity
- 15, 17, 18, 19: Nonwoven sheet
- 16: Air permeable sheet
- 50: Main body
- 60: Attachment unit
- 61: Attachment band part
- 63: Attachment unit formation sheet
- 64: Adhesive layer
- 65: Release paper
- 66: Base end portion
- 70: First mold
- 71: Flat surface
- 72: Projection part
- 80: Second mold
- 81: Flat surface
- 82: Concave part
- 91: Skin
- 100: Heating implement
- 120: Main body sheet
- 121: First sheet
- 122: Second sheet
- 123: Joint part
- 124: Accommodation space
- 130: Exothermic element
- 131: First covering sheet
- 132: Second covering sheet
- 133: Exothermic part
- 134: Joint part
- 212a: First stage portion
- 212b: Second stage portion
- 212c: Concave portion
- 212d: Flat portion

## Claims

1. A heating implement (100) comprising:
a sheet-shaped main body sheet (120) comprising an exothermic element; and
a projection part sheet (10) provided on a surface on one side of the main body sheet (120), wherein
the projection part sheet (10) comprises a projection part (12) projecting away from the main body sheet (120) toward the one side,
the projection part (12) has air permeability,
the projection part sheet (10) includes a nonwoven sheet (15),
the nonwoven sheet (15) includes fibers formed of a first resin material, and a binding part formed of a second resin material having a lower melting point than the first resin material and binding together the fibers,
**characterized in that,** assuming that a magnitude of a load when the projection part (12) is pressed at a speed of 3 mm/min in a direction opposite to a projecting direction of the projection part (12) is set to a first axis, and an amount of crush of the projection part (12) is set to a second axis, a profile of a relationship between the load and the amount of crush comprises:
a first region (R1) in which the amount of crush increases as the load increases; and
a second region (R2) located on a side in which a value of the amount of crush on the second axis is larger than a value of the amount of crush on the second axis in the first region (R1) and having a larger increase rate of the amount of crush associated with increase in the load than the first region (R1), and
in a direction of the second axis, a range (L2) of the second region (R2) is wider than a range (L1) of the first region (R1), wherein
the measurement is performed according to JIS K7181,
as the projection part is pressed at the speed of 3 mm/min, a load is applied to the projection part in the direction opposite to the projecting direction of the projection part, and the magnitude of the load and the amount of crush of the projection part are measured, and
a load cell having a capacity of 250 N is used for measuring the magnitude of the load.

2. The heating implement (100) according to claim 1, wherein the profile further comprises a third region (R3) located on a side in which a value of the amount of crush on the second axis is larger than the value of the amount of crush on the second axis in the second region (R2) and having a smaller increase rate of the amount of crush associated with increase in the load than the second region (R2).

3. The heating implement (100) according to claim 2, wherein when the profile is approximated by three polygonal lines continuous with each other, a region corresponding to a first polygonal line portion (R31) is the first region (R1), a region corresponding to a second polygonal line portion (R32) adjacent to the first polygonal line portion is the second region (R2), and a region corresponding to a third polygonal line portion (R33) adjacent to the second polygonal line portion is the third region.

4. The heating implement (100) according to claim 3, wherein the load is 0 at one end of the first region (R1).

5. The heating implement (100) according to any one of claims 3 to 4, wherein in a range in which the load is equal to or less than 100 N, the profile comprises, in the direction of the second axis, a plot point plotted at a plot interval of equal to or more than 1/180 of a height dimension of the projection part (12) and equal to or less than 1/100 of the height dimension of the projection part (12),
of sampling values from a measurement start point to a measurement end point, each of consecutive five sampling values is set to a unit sample group,
of the five sampling values of the unit sample group, a sampling value in which the amount of crush is the third is set to a center sampling value,
for each unit sample group, a slope of an approximate straight line obtained by a least squares method is obtained, and a graph in which the obtained slope and the amount of crush of each center sampling value are plotted in a twodimensional coordinate system is obtained,
in the graph, at a region in which the amount of crush is smaller than the amount of crush at a point corresponding to a boundary point between the third region and the second region (R2), and the amount of crush is larger than the amount of crush at a point corresponding to a boundary point between the second region (R2) and the first region (R1), a plot point having a maximum value of the slope of the approximate straight line is set to a maximum inclination plot point,
and furthermore, when the maximum inclination plot point is used as a starting point, and evaluation is sequentially performed from plot points corresponding to a unit sample group in which the amount of crush is largest to a side in which the amount of crush is small, a plot point having a first minimum value of the slope of the approximate straight line is set to a minimum plot point, and
assuming that the load at the boundary point between the first region (R1) and the second region (R2) is set to F1, and the load at a point corresponding to the minimum plot point in the profile is set to F2,
0.8 < (F2/F1) ≤ 3 is satisfied.

6. The heating implement (100) according to any one of claims 1 to 5, wherein the second region (R2) comprises a point in which the amount of crush is 1/4 of a height of the projection part (12).

7. The heating implement (100) according to any one of claims 1 to 6, wherein the load at a boundary between the first region (R1) and the second region (R2) is equal to or less than 20 N.

8. The heating implement (100) according to any one of claims 1 to 7, wherein a minimum value of the load in the second region (R2) is equal to or more than 0.2 N.

## Patentansprüche

1. Heizgerät (100), umfassend:
einen bogenförmigen Hauptkörperbogen (120), der ein exothermes Element umfasst; und
einen Fortsatzteilbogen (10), der auf einer Oberfläche an einer Seite des Hauptkörperbogens (120) bereitgestellt ist, wobei
der Fortsatzteilbogen (10) ein Fortsatzteil (12) umfasst, das sich von dem Hauptkörperbogen (120) weg zu der einen Seite fortsetzt,
das Fortsatzteil (12) Luftdurchlässigkeit aufweist,
der Fortsatzteilbogen (10) einen Vliesstoffbogen (15) beinhaltet,
der Vliesstoffbogen (15) aus einem ersten Harzmaterial gebildete Fasern und ein Bindungsteil beinhaltet, das aus einem zweiten Harzmaterial, das einen niedrigeren Schmelzpunkt als das erste Harzmaterial aufweist, gebildet ist und die Fasern aneinander bindet,
**dadurch gekennzeichnet, dass**
angenommen, dass eine Größe einer Last, wenn das Fortsatzteil (12) bei einer Geschwindigkeit von 3 mm/min in einer Richtung entgegen einer Fortsatzrichtung des Fortsatzteils (12) gepresst wird, auf eine erste Achse festgesetzt wird, und ein Knautschausmaß des Fortsatzteils (12) auf eine zweite Achse festgesetzt wird, ein Profil einer Beziehung zwischen der Last und dem Knautschausmaß Folgendes umfasst:
ein erstes Gebiet (R1), in dem das Knautschausmaß zunimmt, wenn die Last zunimmt; und
ein zweites Gebiet (R2), das auf einer Seite liegt, in der ein Wert des Knautschausmaßes auf der zweiten Achse größer als ein Wert des Knautschausmaßes auf der zweiten Achse in dem ersten Gebiet (R1) ist und das eine größere Zunahmerate des Knautschausmaßes, das mit der Zunahme der Last verknüpft ist, als das erste Gebiet (R1) aufweist, und
in einer Richtung der zweiten Achse eine Reichweite (L2) des zweiten Gebiets (R2) weiter als eine Reichweite (L1) des ersten Gebiets (R1) ist, wobei
die Messung gemäß JIS K7181 durchgeführt wird,
wenn das Fortsatzteil bei der Geschwindigkeit von 3 mm/min gepresst wird, eine Last auf das Fortsatzteil in der Richtung entgegen der Fortsatzrichtung des Fortsatzteils angewendet wird und das Ausmaß der Last und das Knautschausmaß des Fortsatzteils gemessen werden, und
eine Lastzelle, die eine Kapazität von 250 N aufweist, zum Messen des Ausmaßes der Last verwendet wird.

2. Heizgerät (100) nach Anspruch 1, wobei das Profil weiter ein drittes Gebiet (R3) umfasst, das auf einer Seite liegt, in der ein Wert des Knautschausmaßes auf der zweiten Achse größer als der Wert des Knautschausmaßes auf der zweiten Achse in dem zweiten Gebiet (R2) ist und das eine kleinere Zunahmerate des Knautschausmaßes, das mit Zunahme der Last verknüpft ist, als das zweite Gebiet (R2) aufweist.

3. Heizgerät (100) nach Anspruch 2, wobei, wenn das Profil von drei polygonalen Linien, die miteinander fortlaufend sind, angenähert wird, ein Gebiet, das einem ersten polygonalen Linienabschnitt (R31) entspricht, das erste Gebiet (R1) ist, ein Gebiet, das einem zweiten polygonalen Linienabschnitt (R32) entspricht, der an den ersten polygonalen Linienabschnitt angrenzt, das zweite Gebiet (R2) ist, und ein Gebiet, das einem dritten polygonalen Linienabschnitt (R33) entspricht, der an den zweiten polygonalen Linienabschnitt angrenzt, das dritte Gebiet ist.

4. Heizgerät (100) nach Anspruch 3, wobei die Last an einem Ende des ersten Gebiets (R1) 0 ist.

5. Heizgerät (100) nach einem der Ansprüche 3 bis 4, wobei in einer Reichweite, in der die Last gleich oder kleiner als 100 N ist, das Profil in der Richtung der zweiten Achse einen Plotpunkt umfasst, der bei einem Plotintervall gleich oder größer als 1/180 einer Höhenabmessung des Fortsatzteils (12) und gleich oder kleiner als 1/100 der Höhenabmessung des Fortsatzteils (12) geplottet wird,
von Abtastungswerten von einem Messungsstartpunkt zu einem Messungsendpunkt jeder von aufeinanderfolgenden fünf Abtastungswerten für eine Einheitsabtastungsgruppe festgesetzt wird,
von den fünf Abtastungswerten der Einheitsabtastungsgruppe ein Abtastungswert, in dem das Knautschausmaß das dritte ist, für einen mittleren Abtastungswert festgesetzt wird,
für jede Einheitsabtastungsgruppe eine Steigung einer annähernd geraden Linie erhalten wird, die durch eine Methode kleinster Quadrate erhalten wird, und ein Graph, in dem die erhaltene Steigung und das Knautschausmaß jedes mittleren Abtastungswerts in einem zweidimensionalen Koordinatensystem geplottet werden, erhalten wird,
in dem Graphen, bei einem Gebiet, in dem das Knautschausmaß kleiner als das Knautschausmaß bei einem Punkt, der einem Grenzpunkt zwischen dem dritten Gebiet und dem zweiten Gebiet (R2) entspricht, ist und das Knautschausmaß größer als das Knautschausmaß bei einem Punkt, der einem Grenzpunkt zwischen dem zweiten Gebiet (R2) und dem ersten Gebiet (R1) entspricht, ist, ein Plotpunkt, der einen maximalen Wert der Steigung der annähernd geraden Linie aufweist, auf einen maximalen Anstiegsplotpunkt festgesetzt wird,
und darüber hinaus, wenn der maximale Anstiegsplotpunkt als ein Startpunkt verwendet wird, und Evaluierung nacheinander von Plotpunkten, die einer Einheitsabtastungsgruppe entsprechen, in der das Knautschausmaß am größten ist, zu einer Seite, in der das Knautschausmaß klein ist, durchgeführt wird, ein Plotpunkt, der einen ersten minimalen Wert der Steigung der annähernd geraden Linie aufweist, auf einen minimalen Plotpunkt festgesetzt wird, und
angenommen, dass die Last bei dem Grenzpunkt zwischen dem ersten Gebiet (R1) und dem zweiten Gebiet (R2) auf F1 festgesetzt wird und die Last bei einem Punkt, der dem minimalen Plotpunkt in dem Profil entspricht, auf F2 festgesetzt wird,
0,8 < (F2/F1) ≤ 3 erfüllt ist.

6. Heizgerät (100) nach einem der Ansprüche 1 bis 5, wobei das zweite Gebiet (R2) einen Punkt umfasst, in dem das Knautschausmaß 1/4 einer Höhe des Fortsatzteils (12) ist.

7. Heizgerät (100) nach einem der Ansprüche 1 bis 6, wobei die Last bei einer Grenze zwischen dem ersten Gebiet (R1) und dem zweiten Gebiet (R2) gleich oder kleiner als 20 N ist.

8. Heizgerät (100) nach einem der Ansprüche 1 bis 7, wobei ein minimaler Wert der Last in dem zweiten Gebiet (R2) gleich oder mehr als 0,2 N ist.

## Revendications

1. Instrument de chauffage (100) comprenant :
une feuille de corps principal en forme de feuille (120) comprenant un élément exothermique ; et
une feuille de partie en saillie (10) fournie sur une surface d'un côté de la feuille de corps principal (120), dans lequel
la feuille de partie en saillie (10) comprend une partie en saillie (12) faisant saillie vers l'extérieur depuis la feuille de corps principal (120) vers le un côté,
la partie en saillie (12) présente une perméabilité à l'air,
la feuille de partie en saillie (10) inclut une feuille non tissée (15),
la feuille non tissée (15) inclut des fibres formées à partir d'un premier matériau de résine, et une partie de liaison formée à partir d'un second matériau de résine présentant un point de fusion inférieur au premier matériau de résine et reliant ensemble les fibres,
**caractérisé en ce que**,
en supposant qu'une ampleur d'une charge lorsque la partie en saillie (12) est pressée à une vitesse de 3 mm/min dans une direction opposée à une direction de saillie de la partie en saillie (12) est définie sur un premier axe, et qu'une quantité de broyage de la partie en saillie (12) est définie sur un second axe, un profil d'une relation entre la charge et la quantité de broyage comprend :
une première région (R1) dans laquelle la quantité de broyage augmente tandis que la charge augmente ; et
une deuxième région (R2) située sur un côté dans laquelle une valeur de la quantité de broyage sur le second axe est supérieure à une valeur de la quantité de broyage sur le second axe dans la première région (R1) et présentant un taux d'augmentation de la quantité de broyage associé à l'augmentation dans la charge supérieur à la première région (R1), et
dans une direction du second axe, une plage (L2) de la deuxième région (R2) est plus large qu'une plage (L1) de la première région (R1), dans lequel
la mesure est effectuée conformément à JIS K7181,
tandis que la partie en saillie est pressée à la vitesse de 3 mm/min, une charge est appliquée à la partie en saillie dans la direction opposée à la direction de saillie de la partie en saillie, et l'ampleur de la charge et la quantité de broyage de la partie en saillie sont mesurées, et
une cellule de charge présentant une capacité de 250 N est utilisée pour mesurer l'ampleur de la charge.

2. Instrument de chauffage (100) selon la revendication 1, dans lequel le profil comprend en outre une troisième région (R3) située sur un côté dans laquelle une valeur de la quantité de broyage sur le second axe est supérieure à la valeur de la quantité de broyage sur le second axe dans la deuxième région (R2) et présentant un taux d'augmentation de la quantité de broyage associé à l'augmentation dans la charge inférieur à la deuxième région (R2).

3. Instrument de chauffage (100) selon la revendication 2, dans lequel, lorsque le profil est approché par trois lignes polygonales continues les unes avec les autres, une région correspondant à une première portion de ligne polygonale (R31) est la première région (R1), une région correspondant à une deuxième portion de ligne polygonale (R32) adjacente à la première portion de ligne polygonale est la deuxième région (R2), et une région correspondant à une troisième portion de ligne polygonale (R33) adjacente à la deuxième portion de ligne polygonale est la troisième région.

4. Instrument de chauffage (100) selon la revendication 3, dans lequel la charge est 0 au niveau d'une extrémité de la première région (R1).

5. Instrument de chauffage (100) selon l'une quelconque des revendications 3 à 4, dans lequel, dans une plage dans laquelle la charge est égale ou inférieure à 100 N, le profil comprend, dans la direction du second axe, un point de traçage tracé au niveau d'un intervalle de traçage égal ou supérieur à 1/180 d'une dimension de hauteur de la partie en saillie (12) et égal ou inférieur à 1/100 de la dimension de hauteur de la partie en saillie (12),
sur des valeurs d'échantillonnage depuis un point de départ de mesure jusqu'à un point de fin de mesure, chacune de cinq valeurs d'échantillonnage consécutives est définie sur un groupe d'échantillons unitaires,
sur les cinq valeurs d'échantillonnage du groupe d'échantillons unitaires, une valeur d'échantillonnage dans laquelle la quantité de broyage est la troisième est définie sur une valeur d'échantillonnage centrale,
pour chaque groupe d'échantillons unitaires, une pente d'une ligne droite approximative obtenue par une méthode des moindres carrés est obtenue, et un graphique dans lequel la pente obtenue et la quantité de broyage de chaque valeur d'échantillonnage centrale sont tracées dans un système de coordonnées bidimensionnel est obtenu,
dans le graphique, au niveau d'une région dans laquelle la quantité de broyage est inférieure à la quantité de broyage au niveau d'un point correspondant à un point de limite entre la troisième région et la deuxième région (R2), et la quantité de broyage est supérieure à la quantité de broyage au niveau d'un point correspondant à un point de limite entre la deuxième région (R2) et la première région (R1), un point de traçage présentant une valeur maximale de la pente de la ligne droite approximative est défini sur un point de traçage d'inclinaison maximale,
et en outre, lorsque le point de traçage d'inclinaison maximale est utilisé comme point de départ, et une évaluation est effectuée séquentiellement à partir de points de traçage correspondant à un groupe d'échantillons unitaires dans lequel la quantité de broyage est la plus grande jusqu'à un côté dans lequel la quantité de broyage est petite, un point de traçage présentant une première valeur minimale de la pente de la ligne droite approximative est défini sur un point de traçage minimal, et
en supposant que la charge au niveau du point de limite entre la première région (R1) et la deuxième région (R2) est définie sur F1, et que la charge au niveau d'un point correspondant au point de traçage minimal dans le profil est définie sur F2,
0,8 < (F2 / F1) ≤ 3 est satisfait.

6. Instrument de chauffage (100) selon l'une quelconque des revendications 1 à 5, dans lequel la deuxième région (R2) comprend un point dans lequel la quantité de broyage représente 1/4 d'une hauteur de la partie en saillie (12).

7. Instrument de chauffage (100) selon l'une quelconque des revendications 1 à 6, dans lequel la charge au niveau d'une limite entre la première région (R1) et la deuxième région (R2) est égale ou inférieure à 20 N.

8. Instrument de chauffage (100) selon l'une quelconque des revendications 1 à 7, dans lequel une valeur minimale de la charge dans la deuxième région (R2) est égale ou supérieure à 0,2 N.
